(19) Europäisches Patentamt
European Patent Office
Office européen des brevets

(11) EP 3 731 291 A1

(12) **EUROPEAN PATENT APPLICATION**
published in accordance with Art. 153(4) EPC

(43) Date of publication:
**28.10.2020 Bulletin 2020/44**

(21) Application number: **18891388.3**

(22) Date of filing: **19.12.2018**

(51) Int Cl.:
***H01L 51/50*** *(2006.01)* ***C09D 7/61*** *(2018.01)*
***C09D 7/63*** *(2018.01)* ***C09D 179/02*** *(2006.01)*
***H05B 33/10*** *(2006.01)*

(86) International application number:
**PCT/JP2018/046690**

(87) International publication number:
**WO 2019/124413 (27.06.2019 Gazette 2019/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2017 JP 2017244506**
**17.07.2018 JP 2018134156**

(71) Applicant: **Nissan Chemical Corporation**
**Tokyo 103-6119 (JP)**

(72) Inventors:
- **MAEDA, Daisuke**
  **Funabashi-shi, Chiba 274-0052 (JP)**
- **KURATA, Yosuke**
  **Funabashi-shi, Chiba 274-0052 (JP)**

(74) Representative: **Mewburn Ellis LLP**
**Aurora Building**
**Counterslip**
**Bristol BS1 6BX (GB)**

(54) **CHARGE-TRANSPORTING VARNISH AND CHARGE-TRANSPORTING THIN FILM**

(57) Disclosed are: a charge transporting varnish containing an oligoaniline compound, a specific sulfonic acid ester compound, metal oxide nanoparticles, and an organic solvent; a charge transporting thin film produced from the charge transporting varnish; an electronic device and an organic electroluminescent device having the charge transporting thin film; a method for producing the charge transporting thin film using the charge transporting varnish; a method for producing the charge transporting varnish; and a method for improving the storage stability of the charge transporting varnish.

EP 3 731 291 A1

## Description

## TECHNICAL FIELD

The present invention relates to a charge transporting varnish and a charge transporting thin film.

## BACKGROUND ART

Charge transporting thin films made of organic compounds are used as the light-emitting layer and the charge injection layer in organic electroluminescent (EL) devices. In particular, the hole injection layer is responsible for transferring charge between the anode and the hole transport layer or the light-emitting layer, and thus serves important functions in achieving low-voltage driving and high brightness in organic EL devices.

In recent years, oligoaniline compounds have been used as the charge transporting material in the hole injection layer, and efforts are being made to improve various characteristics of these compounds. Examples of such characteristics include charge transportability, and the luminous efficacy and luminance characteristics exhibited when the compounds are used in an organic EL device, as well as solubility, in particular in an organic solvent, for compatibility with various coating methods such as spin coating, ink jet coating, spray coating, and the like that are used when applying a coating of a charge transporting varnish comprising these compounds to form a hole injection layer.

In fact, charge transporting varnishes composed of a homogeneous solution of such an oligoaniline compound dissolved in an organic solvent have been found and it has been reported that an underlying substrate planarization effect and excellent organic EL device characteristics can be obtained by inserting a hole injection layer obtained from such a varnish in an organic EL device (Patent Documents 1 to 3).

At the same time, it has been reported that the driving voltage of an organic EL device can be lowered by using, in combination with an oligoaniline compound, a 1,4-benzodioxanesulfonic acid compound (Patent Document 4) as an electron accepting material.

Since sulfonic acid compounds have poor solubility in low polarity organic solvents, however, preparing a solution of a sulfonic acid compound requires, unless it is an aqueous solution, use of a solvent comprising a high polarity organic solvent having high solvency, such as N,N-dimethylacetamide and N-methylpyrrolidone. A solution comprising a highly polar organic solvent may cause damage to part of an inkjet coating apparatus or organic structures formed on a substrate such as an insulating film and a bank. Another problem is that, if a charge transporting varnish comprising a highly polar organic solvent is subjected to prolonged atmospheric exposure, it results in an increase in electrical conductivity over time due to water absorption, thereby compromising inkjet discharge stability. In addition, because of their high polarity, sulfonic acid compounds were difficult to purify by extraction, water rinsing, silica gel column chromatography, and the like.

By contrast, sulfonic acid ester compounds are soluble in various organic solvents, and generate a sulfonic acid compound, which is an organic strong acid, under external stimulation such as heating and chemical action. Therefore, it is believed that a sulfonic acid ester compound can be used as a precursor to a sulfonic acid compound used as an electron accepting substance. The cyclohexyl esters of sulfonic acids and the like have been reported as specific examples of compounds that, on heating, give sulfonic acids (Non-Patent Document 1). Sulfonic acid ester compounds have also attracted attention in connection with the concept of thermal acid generators (Patent Document 5, Non-Patent Document 2).

However, some sulfonic acid ester compounds are unstable and unexpectedly decompose to sulfonic acid compounds. In particular, compounds having a structure in which a sulfonic acid ester structure is bonded to an aromatic ring depleted of electrons (for example, an aromatic disulfonic acid ester or the like) are easily decomposed by slight heat or by reaction with water, a basic substance or the like. The stability of such sulfonic acid ester compounds was insufficient as a component of a charge transporting varnish.

For this reason, there has been a need for a charge transporting varnish for forming the hole injection layer of an organic EL device, comprising, as a precursor of an electron accepting substance, a sulfonic acid ester compound having improved stability.

In order to solve the above problems, the present inventors have reported sulfonic acid ester compounds having high stability as well as high solubility in a wide range of organic solvents (Patent Document 6). The sulfonic acid ester compounds were superior in stability and solubility in organic solvents to sulfonic acid compounds and conventional sulfonic acid ester compounds. There is room for improvement in stability and solubility in an organic solvent, however, because in order to dissolve these compounds in a low polarity solvent, it is necessary to stir at high temperatures for a long time, and the solution may precipitate when stored for a long time.

## PRIOR ART DOCUMENTS

### Patent Documents

Patent Document 1: Japanese Unexamined Patent Application Publication No. 2002-151272
Patent Document 2: WO 2004/043117
Patent Document 3: WO 2005/043962
Patent Document 4: WO 2005/000832
Patent Document 5: Japanese Unexamined Patent Application Publication No. H07-134416
Patent Document 6: Japanese Patent No. 5136795

### Non-Patent Documents

Non-Patent Document 1: Chemische Berichte, 90, pp. 585-592 (1957)
Non-Patent Document 2: Kino Zairyo, 24, pp. 72-82 (2004)

## SUMMARY

### Problems to be Solved by the Invention

The present invention has been made in view of such circumstances. Accordingly, it is an object of the present invention to provide a charge transporting varnish for forming a hole injection layer of an organic EL device, comprising, as a precursor of an electron accepting substance, a sulfonic acid ester compound having improved stability and a charge transporting thin film formed using the same.

### Means for Solving the Problems

The present inventors carried out intensive research in order to solve the problems discussed above. As a result, it was surprisingly found that problems arising due to the solubility of the precursor in a low polarity organic solvent can be avoided in the preparation of a charge transporting varnish by using, as a precursor of an electron accepting substance, a sulfonic acid ester compound that is an ester of a specific sulfonic acid compound and a specific glycol ether compound, and that storing this charge transporting varnish for a long time does not cause problems such as the precipitation of a constituent. The present invention was completed based on the new findings above.
That is, the present invention provides the following.

1. A charge transporting varnish comprising (a) to (d) below:

(a) an oligoaniline compound;
(b) a sulfonic acid ester compound represented by formula (2) below:

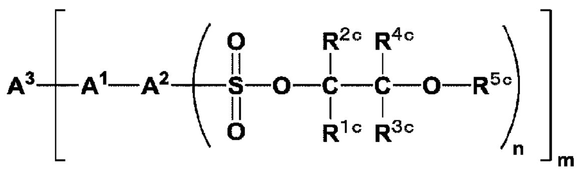

(2)

wherein

$R^{1c}$ to $R^{4c}$ each, independently, represent a hydrogen atom or a linear or branched alkyl group having 1 to 6 carbon atoms,
$R^{5c}$ represents an optionally substituted monovalent hydrocarbon group having 2 to 20 carbon atoms,
$A^1$ represents -O- or -S-,
$A^2$ represents an (n+1)-valent group derived from naphthalene or anthracene,

$A^3$ represents an m-valent group derived from perfluorobiphenyl,
m represents an integer satisfying $2 \leq m \leq 4$, and
n represents an integer satisfying $1 \leq n \leq 4$;

(c) one or more metal oxide nanoparticles; and
(d) an organic solvent.

2. The charge transporting varnish according to preceding item 1, wherein the oligoaniline compound (a) is at least one selected from the group consisting of (i) to (v) below:

(i) an oligoaniline compound represented by formula (1a) below:

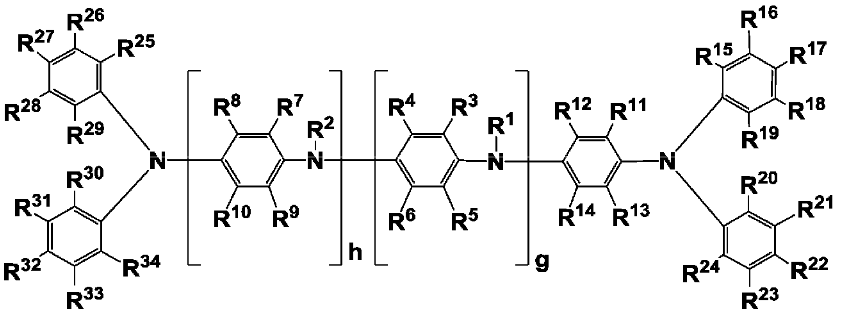 (1a)

wherein

$R^1$ and $R^2$ each, independently, represent a hydrogen atom, a substituted or unsubstituted monovalent hydrocarbon group, a t-butoxycarbonyl group, or a benzyloxycarbonyl group;
$R^3$ to $R^{34}$ each, independently, represent a hydrogen atom, a hydroxyl group, a silanol group, a thiol group, a carboxyl group, a phosphoric acid group, a phosphate ester group, an ester group, a thioester group, an amide group, a nitro group, a substituted or unsubstituted monovalent hydrocarbon group, an organoxy group, an organoamino group, an organosilyl group, an organothio group, an acyl group, a sulfo group or a halogen atom; and
g and h each, independently, represent an integer of 1 or more and satisfy $g + h \leq 20$;

(ii) an oligoaniline compound represented by formula (1b) below:

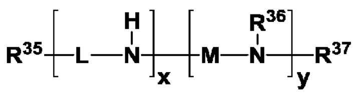 (1b)

wherein

$R^{35}$, $R^{36}$, and $R^{37}$ each, independently, represent a hydrogen atom, an unsubstituted or substituted monovalent hydrocarbon group, or an organoxy group;
L and M each, independently, represent a divalent group represented by formula (b-1) or (b-2) below:

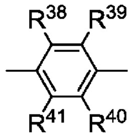 (b-1) 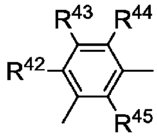 (b-2)

wherein
$R^{38}$ to $R^{45}$ each, independently, represent a hydrogen atom, a hydroxyl group, an unsubstituted or substituted monovalent hydrocarbon group, or an organoxy group, an acyl group, or a sulfonic acid group; and
x and y each, independently, represent an integer of 1 or more, and satisfy $x + y \leq 20$;

(iii) an oligoaniline compound represented by formula (1c) below:

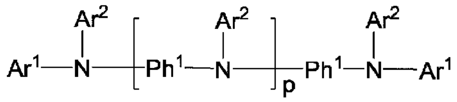

(1c)

wherein
$Ph^1$ is a group represented by formula (PI) below:

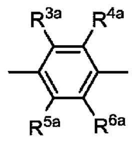

(P1)

wherein

$R^{3a}$ to $R^{6a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^1$ each, independently, represents an unsubstituted or substituted aryl group or heteroaryl group which may have a monoarylamino group and/or a diarylamino group;
$Ar^2$ each, independently, represents an unsubstituted or substituted aryl group or heteroaryl group which may have a monoarylamino group and/or a diarylamino group; and
p represents an integer of 1 to 10;

(iv) an oligoaniline compound represented by formula (Id) below:

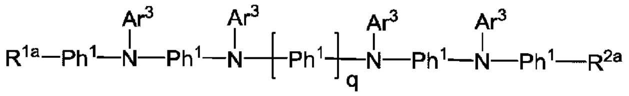

(1d)

wherein

$R^{1a}$ and $R^{2a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^3$ each, independently, represents a diarylaminophenyl group;
q represents 1 or 2; and
$Ph^1$ is the same as defined for formula (1c) above; and

(v) an oligoaniline compound represented by formula (1e) below:

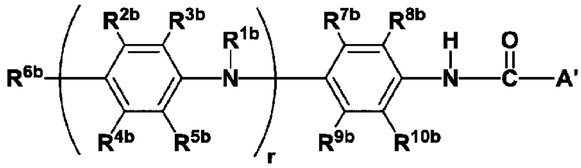

**(1e)**

wherein

$R^{1b}$ represents a hydrogen atom or an alkyl group having 1 to 20 carbon atoms optionally substituted with $Z^b$;
$Z^b$ represents a halogen atom, a nitro group, a cyano group, an aldehyde group, a hydroxyl group, a thiol group, a sulfonic acid group, a carboxyl group, an aryl group having 6 to 20 carbon atoms optionally

substituted with $Z^{b'}$, or a heteroaryl group having 2 to 20 carbon atoms optionally substituted with $Z^{b'}$;
$Z^{b'}$ represents a halogen atom, a nitro group, a cyano group, an aldehyde group, a hydroxyl group, a thiol group, a sulfonic acid group or a carboxyl group;
$R^{2b}$ to $R^{10b}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
A' represents a hydrocarbon group having 1 to 40 carbon atoms, wherein at least one hydrogen atom is substituted with a fluorine atom and at least one other hydrogen atom is substituted with another atom or substituent; and
r is an integer from 1 to 20.

3. The charge transporting varnish according to preceding item 1 or 2, further comprising at least one silane compound.
4. A charge transporting thin film produced from the charge transporting varnish according to any one of preceding items 1 to 3.
5. An organic electroluminescent device comprising the charge transporting thin film according to preceding item 4.
6. A method for producing a charge transporting thin film, comprising the step of applying a charge transporting varnish according to any one of preceding items 1 to 3 onto a substrate and evaporating the solvent.
7. A method for producing the charge transporting varnish according to preceding item 1, wherein a sol of the one or more metal oxide nanoparticles (c) is used.
8. A method for improving the storage stability of a charge transporting varnish comprising an oligoaniline compound, one or more metal oxide nanoparticles, a precursor of an electron accepting substance, and an organic solvent, wherein a sulfonic acid ester compound represented by formula (2) below is used as a precursor of the electron accepting substance.

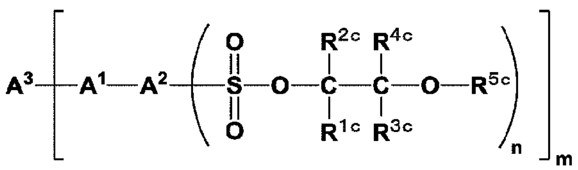

(2)

wherein

$R^{1c}$ to $R^{4c}$ each, independently, represent a hydrogen atom or a linear or branched alkyl group having 1 to 6 carbon atoms,
$R^{5c}$ represents an optionally substituted monovalent hydrocarbon group having 2 to 20 carbon atoms,
$A^1$ represents -O- or -S-,
$A^2$ represents an (n+1)-valent group derived from naphthalene or anthracene,
$A^3$ represents an m-valent group derived from perfluorobiphenyl,
m represents an integer satisfying $2 \leq m \leq 4$, and
n represents an integer satisfying $1 \leq n \leq 4$.

## Effect of the Invention

The charge transporting varnish of the present invention comprises a stable sulfonic acid ester compound having high solubility in low polarity organic solvents that serves as a precursor of an electron accepting substance. Therefore, the varnish can be easily prepared by methods that do not involve troublesome steps such as stirring at a high temperature for a long time, and at the same time, the varnish has improved characteristics without concomitant problems such as the precipitation of a constituent and can be stored for a long time. In addition, the charge transporting varnish of the present invention does not require that a high polarity organic solvent, which may cause damage to an inkjet coating apparatus, organic structures formed on a substrate and the like, be added in order to dissolve an electron accepting substance. Accordingly, the use of the charge transporting varnish of the present invention allows a remarkable reduction of restrictions that are related to the step of applying the charge transporting varnish and arise from the need to avoid the damage discussed above, such as those on application methods and application patterns.
Further, not only is the charge transporting varnish of the present invention suitably used for forming a hole injection layer of an organic EL device as described above, but it can also be used for forming a charge transporting

thin film for use in other electronic devices such as an organic photoelectric conversion device, an organic thin-film solar cell, an organic perovskite photoelectric conversion device, an organic integrated circuit, an organic field effect transistor, an organic thin film transistor, an organic light emitting transistor, an organic optical detector, an organic photoreceptor, an organic field quench device, a light emitting electrochemical cell, a quantum dot light emitting diode, a quantum laser, an organic laser diode and an organic plasmon emitting device.

**Brief Description of the Drawings**

FIG. 1 is a graph showing the light transmittance in the visible region of thin films formed on quartz substrates using the charge transporting varnishes of Examples 2-1 to 2-3 and Comparative Examples 2-1 to 2-2.

**Description of Embodiments**

Hereinafter, the present invention will be described in more detail.
The charge transporting varnish of the present invention comprises:

(a) an oligoaniline compound;
(b) a specific sulfonic acid ester compound;
(c) one or more metal oxide nanoparticles; and
(d) an organic solvent.

Each of these components will be described below.

< Oligoaniline compound (a) >

The oligoaniline compound (a) contained in the charge transporting varnish of the present invention is a compound composed of a plurality of structural units, which may be the same or different, derived from aniline derivatives, having a weight average molecular weight of 200 to 5,000. In the oligoaniline compound (a), two adjacent structural units are bonded to each other. When the oligoaniline compound (a) comprises two or more different structural units, the structural units may be arranged in any order.
In addition, from the perspective of obtaining a highly homogeneous charge transporting varnish and obtaining a charge transporting thin film having excellent flatness reproducibly, in one preferred embodiment, either a single type of oligoaniline compound having a molecular weight within the range of 200 to 5,000 and having no molecular weight distribution is used for the oligoaniline compound (a), or two or three types of oligoaniline compounds each satisfying such conditions are used in combination.
In the present invention, any oligoaniline compound may be used as a charge transporting substance, but, preferably, the oligoaniline compound (a) is at least one oligoaniline compound selected from the group consisting of the oligoaniline compounds (i) to (v) described in sequence below.

- Oligoaniline compound (i)

The oligoaniline compound (i) that can be used as the oligoaniline compound (a) is an oligoaniline compound represented by formula (1a) below:

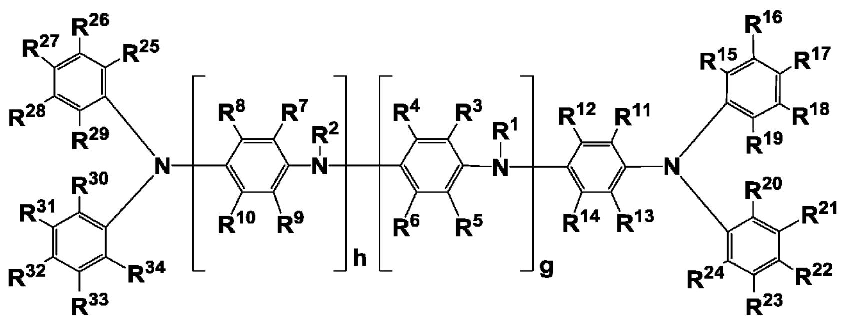

(1a)

wherein

$R^1$ and $R^2$ each, independently, represent a hydrogen atom, a substituted or unsubstituted monovalent hydrocarbon group, a t-butoxycarbonyl group, or a benzyloxycarbonyl group;
$R^3$ to $R^{34}$ each, independently, represent a hydrogen atom, a hydroxyl group, a silanol group, a thiol group, a carboxyl group, a phosphoric acid group, a phosphate ester group, an ester group, a thioester group, an amide group, a nitro group, a substituted or unsubstituted monovalent hydrocarbon group, an organoxy group, an organoamino group, an organosilyl group, an organothio group, an acyl group, a sulfo group or a halogen atom; and
g and h each, independently, represent an integer of 1 or more and satisfy $g + h \leq 20$.

The monovalent hydrocarbon group mentioned above is not specifically restricted in carbon number; however, the monovalent hydrocarbon should preferably have 1 to 20 carbon atoms, more preferably 1 to 8 carbon atoms.

Examples of the substituted or unsubstituted monovalent hydrocarbon group include alkyl groups, such as a methyl group, an ethyl group, an n-propyl group, an i-propyl group, an n-butyl group, an i-butyl group, a t-butyl group, an n-hexyl group, an n-octyl group, a 2-ethylhexyl group, and a decyl group; cycloalkyl groups, such as a cyclopentyl group and a cyclohexyl group; bicycloalkyl groups, such as a bicyclohexyl group; alkenyl groups, such as a vinyl group, a 1-propenyl group, a 2-propenyl group, a 1-methyl-2-propenyl group, a 1- or 2- or 3-butenyl group, and a hexenyl group; aryl groups, such as a phenyl group, a xylyl group, a tolyl group, a biphenyl group, and a naphthyl group; and aralkyl groups, such as a benzyl group, a phenylethyl group, and a phenylcyclohexyl group. These monovalent hydrocarbon groups may have their hydrogen atoms partly or entirely substituted with halogen atoms, hydroxyl groups, alkoxyl groups, sulfo groups, or the like.

It should be noted that "unsubstituted" means bonding of hydrogen atoms. Also, substituent groups may link with each other to form a cyclic structure.

$R^1$ and $R^2$ above each, independently, are, preferably, a hydrogen atom, a methyl group, an ethyl group, or a t-butoxycarbonyl group, of which a hydrogen atom or a t-butoxycarbonyl group is particularly preferable. In other words, preferred embodiments are those in which both $R^1$ and $R^2$ are hydrogen atoms or t-butoxycarbonyl groups, $R^1$ is a hydrogen atom and $R^2$ is a t-butoxycarbonyl group, or $R^1$ is a t-butoxycarbonyl group and $R^2$ is a hydrogen atom.

In formula (1a) above, $R^3$ to $R^{34}$ each, independently, represent a hydrogen atom, a hydroxyl group, an amino group, a silanol group, a thiol group, a carboxyl group, a phosphoric acid group, a phosphate ester group, an ester group, a thioester group, an amide group, a nitro group, a substituted or unsubstituted monovalent hydrocarbon group, an organoxy group, an organoamino group, an organosilyl group, an organothio group, an acyl group, a sulfo group, a halogen atom, or the like.

Examples of the substituted or unsubstituted monovalent hydrocarbon group are the same as those listed above.

Examples of the organoxy group include an alkoxyl group, an alkenyloxy group, an aryloxy group, and the like. Examples of the alkyl group and the alkenyl group also include the same substituent groups listed above.

Examples of the organoamino group include alkylamino groups, such as a methylamino group, an ethylamino group, a propylamino group, a butylamino group, a pentylamino group, a hexylamino group, a heptylamino group, an octylamino group, a nonylamino group, a decylamino group, and a laurylamino group; dialkylamino groups, such as a dimethylamino group, a diethylamino group, a dipropylamino group, a dibutylamino group, a dipentylamino group, a dihexylamino group, a diheptylamino group, a dioctylamino group, a dinonylamino group, and a didecylamino group; cycloalkylamino groups such as a cyclohexylamino group; dicycloalkylamino groups, such as a dicyclohexylamino group; a morpholino group; and arylamino groups, such as a biphenylamino group.

Examples of the organosilyl group include a trimethylsilyl group, a triethylsilyl group, a tripropylsilyl group, a tributylsilyl group, a tripentylsilyl group, a trihexylsilyl group, a pentyldimethylsilyl group, a hexyldimethylsilyl group, an octyldimethylsilyl group, and a decyldimethylsilyl group.

Examples of the organothio group include alkylthio groups, such as a methylthio group, an ethylthio group, a propylthio group, a butylthio group, a pentylthio group, a hexylthio group, a heptylthio group, an octylthio group, a nonylthio group, a decylthio group, and a laurylthio group.

Examples of the acyl group include a formyl group, an acetyl group, a propionyl group, a butylyl group, an isobutylyl group, a valeryl group, an isovaleryl group, and a benzoyl group.

Examples of the halogen atom include a chlorine atom, a bromine atom, a fluorine atom, and an iodine atom.

The phosphate ester group is exemplified by $-P(O)(OQ^1)(OQ2)$.

The ester group is exemplified by $-C(O)OQ^1$ and $-OC(O)Q^1$.

The thioester group is exemplified by $-C(S)OQ^1$ and $-OC(S)Q^1$.

The amide group is exemplified by $-C(O)NHQ^1$, $-NHC(O)Q^1$, $-C(O)NQ^1Q^2$ and $-NQ^1C(O)Q^2$.

Here, the foregoing $Q^1$ and $Q^2$ denote an alkyl group, an alkenyl group, or an aryl group; examples thereof include the same as those listed for the monovalent hydrocarbon group in the above.

The foregoing monovalent hydrocarbon group, organoxy group, organoamino group, organosilyl group, organothio group, acyl group, phosphate ester group, ester group, thioester group, and amide group denoted by $R^3$ to $R^{34}$ are not specifically restricted in carbon number. However, their carbon number is typically 1 to 20, preferably 1 to 8.

Preferable among these groups, which are denoted by $R^3$ to $R^{34}$ mutually independently, are a hydrogen atom, a substituted or unsubstituted monovalent hydrocarbon group, an organoxy group, an organoamino group, and a halogen atom. Particularly preferable among them are a hydrogen atom, a substituted or unsubstituted monovalent hydrocarbon group, and a halogen atom.

Preferred monovalent hydrocarbon groups are a phenyl group, a biphenyl group, and a naphthyl group.

A preferred halogen atom is a fluorine atom. Preferred organoamino groups are arylamino groups, in particular, diphenylamino groups.

In an embodiment, $R^3$ to $R^{34}$ are all hydrogen atoms.

In formula (1a) above, g and h each, independently, represent an integer of 1 or more, and satisfy $g + h \leq 20$, preferably $g + h \leq 10$, more preferably $g + h \leq 5$.

Adjustment to these ranges allows easy realization of high solubility in various solvents while maintaining good charge transportability.

The oligoaniline compound represented by formula (1a) should preferably have no molecular weight distribution, or, stated differently, should be an oligoaniline compound having a degree of dispersion of 1 for achieving both high solubility and uniform charge transportability.

The lower limit of the molecular weight is typically 200 or higher, preferably 400 or higher, for curving material volatility and achieving good charge transportability. The upper limit is typically 5,000 or lower, preferably 3,000 or lower, for improved solubility.

It should be noted that, in the present invention, a quinone diimine compound that is an oxidized form of the oligoaniline compound represented by formula (1a) above can also be suitably used for the oligoaniline compound (a).

The oligoaniline compound represented by formula (1a) above can be produced, for example, by the method described in WO 2008/129947.

- Oligoaniline compound (ii)

The oligoaniline compound (ii) that can be used as the oligoaniline compound (a) is an oligoaniline compound represented by formula (1b) below:

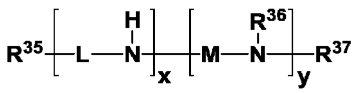 (1b)

wherein

$R^{35}$, $R^{36}$, and $R^{37}$ each, independently, represent a hydrogen atom, an unsubstituted or substituted monovalent hydrocarbon group, or an organoxy group;
L and M each, independently, represent a divalent group represented by formula (b-1) or (b-2) below:

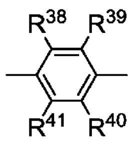 (b-1) 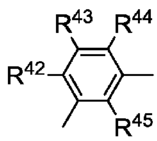 (b-2)

wherein

$R^{38}$ to $R^{45}$ each, independently, represent a hydrogen atom, a hydroxyl group, an unsubstituted or substituted monovalent hydrocarbon group, or an organoxy group, an acyl group, or a sulfonic acid group; and
x and y each, independently, represent an integer of 1 or more, and satisfy $x + y \leq 20$.

$R^{35}$ to $R^{45}$ in formulas (1b) as well as (b-1) and (b-2) are preferably a hydrogen atom from the perspective of ease of synthesis, but an alkyl group, an alkoxy group, a cyclohexyl group, a biphenyl group, a bicyclohexyl group, a phenylcyclohexyl group, and the like are preferable for improving solubility in a solvent.

Typical examples of alkyl groups include a methyl group, an ethyl group, a propyl group, and the like. The number of carbon atoms is typically from 1 to 4, but up to 20 carbon atoms may be introduced.

The values for x and y in formula (1b) above are each, independently, an integer of 1 or more, but 2 or more is preferable in consideration of conductivity, and 20 or less is preferable in consideration of solubility in a solvent.

For $R^{36}$, a hydrogen atom, a hydroxyl group, an unsubstituted or substituted monovalent hydrocarbon group,

an organoxy group, an acyl group, an alkyl group having 1 to 20 carbon atoms, or an alkoxy group having 1 to 20 carbon atoms is suitable. Typical examples of alkyl groups include a methyl group, an ethyl group, a propyl group, and the like. The number of carbon atoms is typically from 1 to 4, but up to 20 carbon atoms may be introduced.

It should be noted that, in the present invention, a quinone diimine compound that is an oxidized form of the oligoaniline compound represented by formula (1b) above can also be suitably used for the oligoaniline compound (a).

- Oligoaniline compound (iii)

The oligoaniline compound (iii) that can be used as the oligoaniline compound (a) is an oligoaniline compound represented by formula (1c) below:

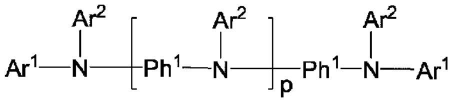

(1c)

wherein
$Ph^1$ is a group represented by formula (PI) below:

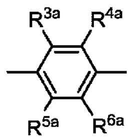

(P1)

wherein

$R^{3a}$ to $R^{6a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^1$ each, independently, represents an unsubstituted or substituted aryl group or heteroaryl group which may have a monoarylamino group and/or a diarylamino group;
$Ar^2$ each, independently, represents an unsubstituted or substituted aryl group or heteroaryl group which may have a monoarylamino group and/or a diarylamino group; and
p represents an integer of 1 to 10.

Examples of halogen atoms include the same as those listed above.

The alkyl group having 1 to 20 carbon atoms may be linear, branched or cyclic. Specific examples thereof include linear or branched alkyl groups having 1 to 20 carbon atoms such as methyl, ethyl, n-propyl, isopropyl, n-butyl, isobutyl, s-butyl, tert-butyl, n-pentyl, n-hexyl, n-heptyl, n-octyl, n-nonyl and n-decyl groups; and cyclic alkyl groups having 3 to 20 carbon atoms such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl groups and the like.

The alkenyl group having 2 to 20 carbon atoms may be linear, branched or cyclic. Specific examples thereof include ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pentenyl, n-1-decenyl and n-1-eicosenyl groups and the like.

The alkynyl group having 2 to 20 carbon atoms may be linear, branched or cyclic. Specific examples thereof include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl groups and the like .

Specific examples of the aryl group having 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl groups.

Specific examples of the heteroaryl group having 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl groups and

the like.

$R^{3a}$ to $R^{6a}$ are preferably hydrogen atoms, fluorine atoms, cyano groups, alkyl groups having 1 to 20 carbon atoms which may be substituted with halogen atoms, aryl groups having 6 to 20 carbon atoms which may be substituted with halogen atoms, or heteroaryl groups having 2 to 20 carbon atoms which may be substituted with halogen atoms; more preferably, hydrogen atoms, fluorine atoms, cyano groups, alkyl groups having 1 to 10 carbon atoms which may be substituted with halogen atoms, or phenyl groups which may be substituted with halogen atoms; even more preferably, hydrogen atoms or fluorine atoms; and most preferably, hydrogen atoms.

Preferred examples of $Ph^1$ include, but are not limited to, the following.

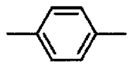

(P1-1)

Preferably, $Ar^1$ each independently represents a group represented by any of formulas (B1) to (B11) below.

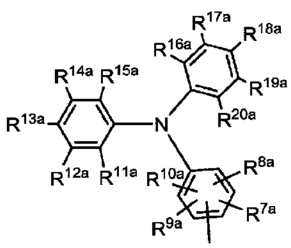

(B1)

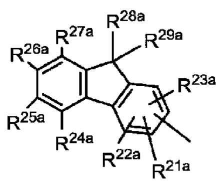

(B2)

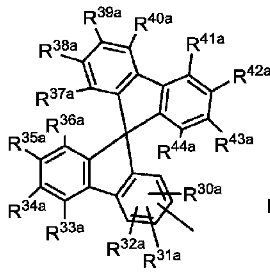

(B3)

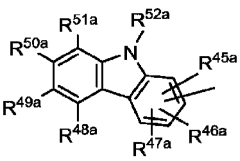

(B4)

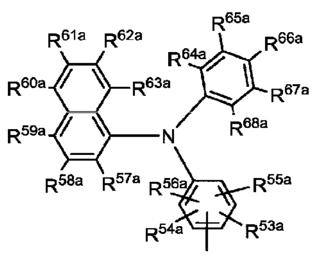

(B5)

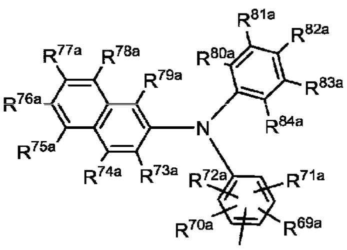

(B6)

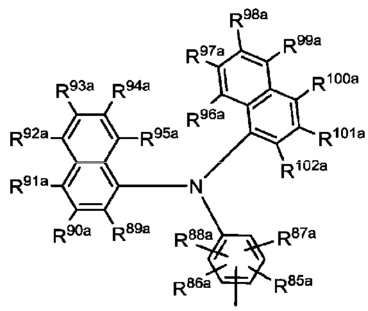

(B7)

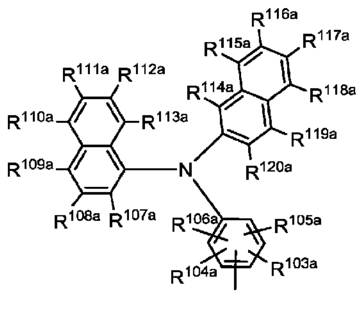

(B8)

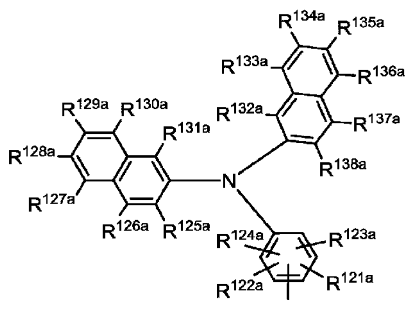

(B9)

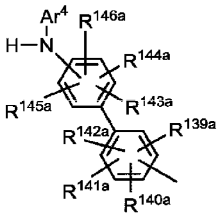

(B10)

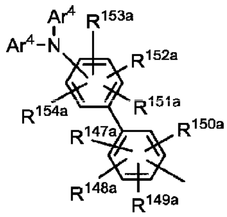

(B11)

wherein

$Ar^4$ each, independently, represents an aryl group having 6 to 20 carbon atoms which may be substituted with a di(C6-20 aryl)amino group;
$R^{7a}$ to $R^{27a}$, $R^{30a}$ to $R^{51a}$ and $R^{53a}$ to $R^{154a}$ each, independently, represent a hydrogen atom, a halogen atom, a nitro group, a cyano group, or a diphenylamino group, an alkyl group having 1 to 20 carbon atoms, an alkenyl group having 2 to 20 carbon atoms, an alkynyl group having 2 to 20 carbon atoms, an aryl group having 6 to 20 carbon atoms or a heteroaryl group having 2 to 20 carbon atoms that may be substituted with a halogen atom;
$R^{28a}$ to $R^{29a}$ each, independently, represent an aryl group having 6 to 20 carbon atoms or a heteroaryl group having 2 to 20 carbon atoms that may be substituted with $Z^{1a}$;
$R^{52a}$ represents a hydrogen atom or an aryl group having 6 to 20 carbon atoms or a heteroaryl group having 2 to 20 carbon atoms that may be substituted with $Z^{1a}$;
$Z^{1a}$ represents a halogen atom, a nitro group, a cyano group, or an alkyl group having 1 to 20 carbon atoms, an alkenyl group having 2 to 20 carbon atoms or an alkynyl group having 2 to 20 carbon atoms that may be substituted with $Z^{2a}$;
$Z^{2a}$ represents a halogen atom, a nitro group, a cyano group, or an aryl group having 6 to 20 carbon atoms or a heteroaryl group having 2 to 20 carbon atoms that may be substituted with $Z^{3a}$; and
$Z^{3a}$ represents a halogen atom, a nitro group or a cyano group.

More preferably, $Ar^1$ each independently represents a group represented by any of formulas (B1') to (B11') below.

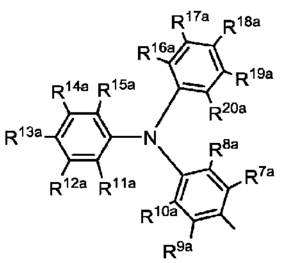

(B1')

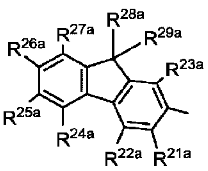

(B2')

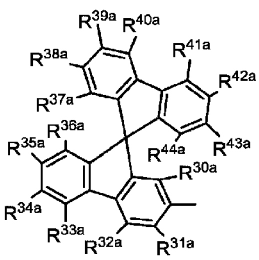

(B3')

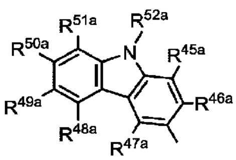

(B4')

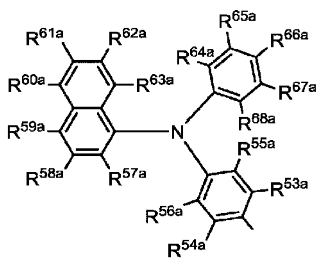

(B5')

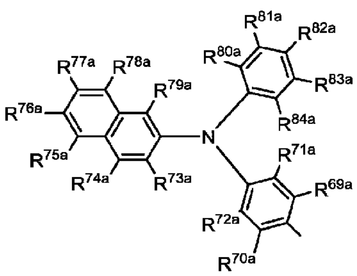

(B6')

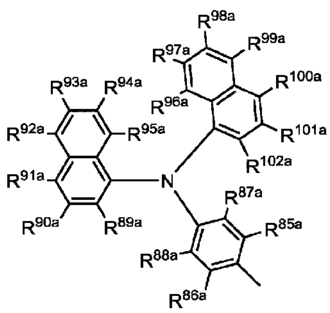

(B7')

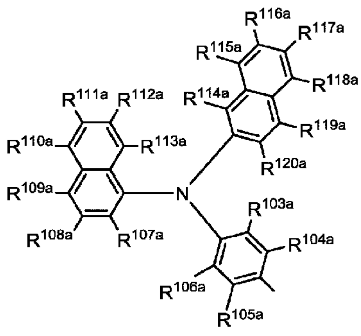
(B8')

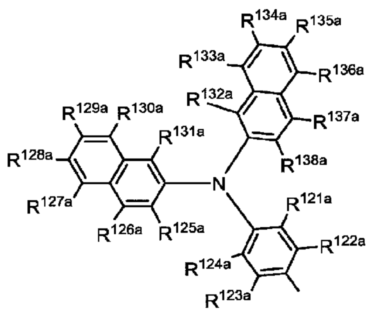
(B9')

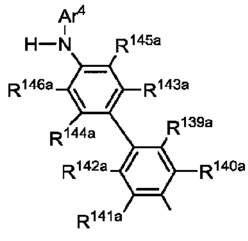
(B10')

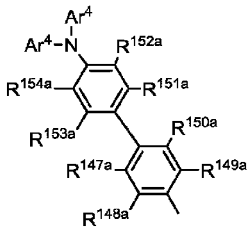
(B11')

wherein $R^{7a}$ to $R^{154a}$ and $Ar^4$ mean the same as above.

Preferably, $Ar^2$ each, independently, represents a group represented by any of formulas (G1) to (G18) below.

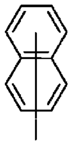
(G1)

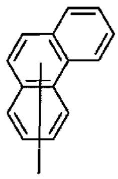
(G2)

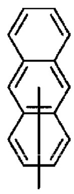
(G3)

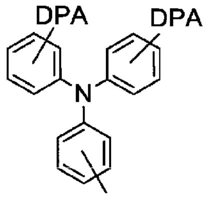
(G4)

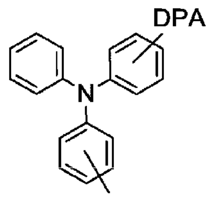
(G5)

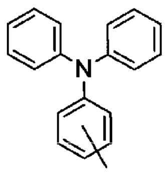
(G6)

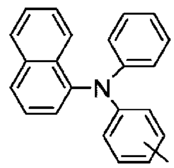
(G7)

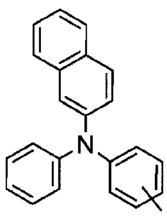
(G8)

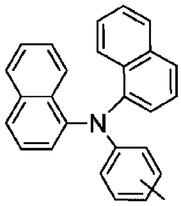
(G9)

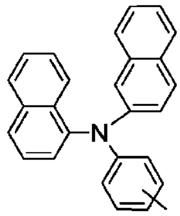
(G10)

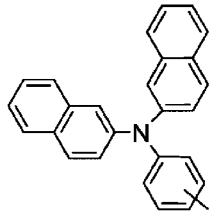
(G11)

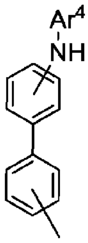
(G12)

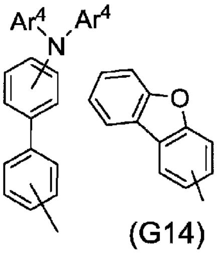
(G13) (G14)

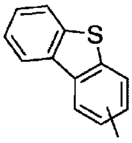
(G15)

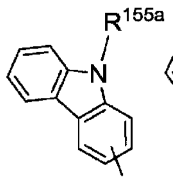
(G16)

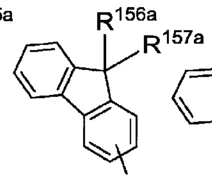
(G17)

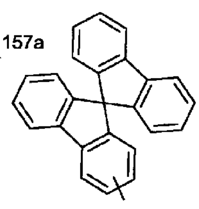
(G18)

wherein

$R^{155a}$ represents a hydrogen atom, an aryl group having 6 to 14 carbon atoms which may be substituted with $Z^{1a}$, or a heteroaryl group having 2 to 14 carbon atoms which may be substituted with $Z^{1a}$;
$R^{156a}$ and $R^{157a}$ each, independently, represent an aryl group having 6 to 14 carbon atoms which may be substituted with a phenyl group that may be substituted with $Z^{1a}$ or a heteroaryl group having 2 to 14 carbon atoms that may be substituted with a phenyl group that may be substituted with $Z^{1a}$;
DPA represents a diphenylamino group; and
$Ar^4$ and $Z^{1a}$ are the same as defined for $Ar^1$.

$R^{155a}$ is preferably a hydrogen atom, an aryl group having 6 to 14 carbon atoms which may be substituted with $Z^{1a}$, or a heteroaryl group having 2 to 14 carbon atoms which may be substituted with $Z^{1a}$; more preferably, a hydrogen atom, a phenyl group which may be substituted with $Z^{1a}$, a 1-naphthyl group which may be substituted with $Z^{1a}$, a 2-naphthyl group which may be substituted with $Z^{1a}$, a 2-pyridyl group which may be substituted with $Z^{1a}$, a 3-pyridyl group which may be substituted with a phenyl group that may be substituted with $Z^{1a}$, or a 4-pyridyl group which may be substituted with $Z^{1a}$; most preferably a phenyl group which may be substituted with $Z^{1a}$; and most preferably, a phenyl group or a 2,3,5,6-tetrafluoro-4-(trifluoromethyl)phenyl group.

$R^{156a}$ and $R^{157a}$ are preferably aryl groups having 6 to 14 carbon atoms which may be substituted with a phenyl group that may be substituted with $Z^{1a}$ or heteroaryl groups having 2 to 14 carbon atoms that may be substituted with a phenyl group that may be substituted with $Z^{1a}$; more preferably, aryl groups having 6 to 14 carbon atoms which may be substituted with a phenyl group that may be substituted with $Z^{1a}$; and even more preferably, phenyl groups which may be substituted with a phenyl group that may be substituted with $Z^{1a}$, 1-naphthyl groups which may be substituted with a phenyl group that may be substituted with $Z^{1a}$ or 2-naphthyl groups which may be substituted with $Z^{1a}$.

Specific examples of the aryl group having 6 to 20 carbon atoms for $Ar^4$ include the same as those mentioned for $R^{3a}$ to $R^{6a}$, and specific examples of the di(C6-20 aryl)amino group include diphenylamino, 1-naphthylphenylamino, di(1-naphthyl)amino, 1-naphthyl-2-naphthylamino and di(2-naphthyl)amino groups and the like.

$Ar^4$ is preferably a phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, p-(diphenylamino)phenyl, p-(1-naphthylphenylamino)phenyl, p-(di(1-naphthyl)amino)phenyl, p-(1-naphthyl-2-naphthylamino)phenyl or p-(di(2-naphthyl)amino)phenyl group; and more preferably, a p-(diphenylamino)phenyl group.

Further, p represents an integer from 1 to 10, and from the perspective of increasing solubility in organic solvents, p is preferably from 1 to 5, more preferably from 1 to 3, even more preferably 1 or 2, and most preferably 1.

More preferably, $Ar^2$ each, independently, represents a group represented by any of formulas (G1') to (G18') below.

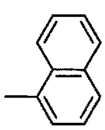
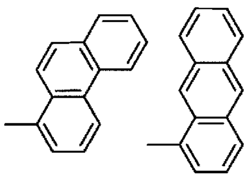
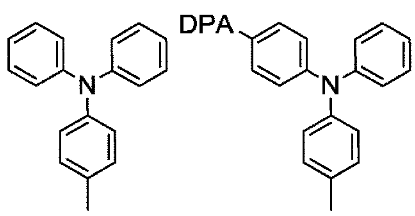
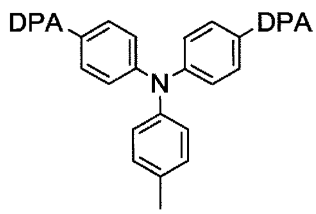

(G1') (G2') (G3') (G4') (G5') (G6')

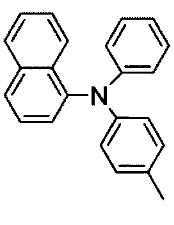
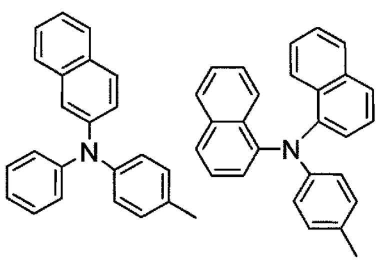
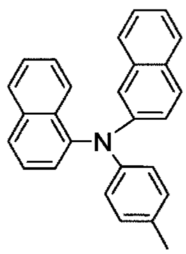
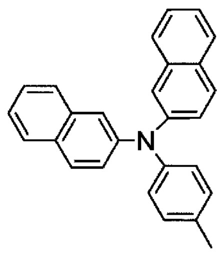

(G7') (G8') (G9') (G10') (G11')

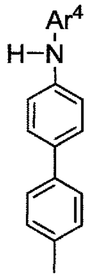 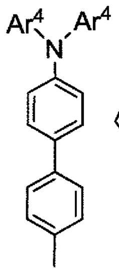 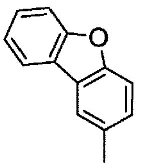 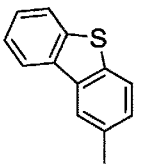 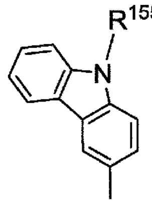 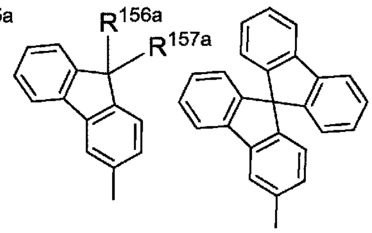

(G12') (G13') (G14') (G15') (G16') (G17') (G18')

wherein $R^{155a}$ to $R^{157a}$, DPA and $Ar^4$ mean the same as above.

- Oligoaniline compound (iv)

The oligoaniline compound (iv) that can be used as the oligoaniline compound (a) is an oligoaniline compound represented by formula (1d) below:

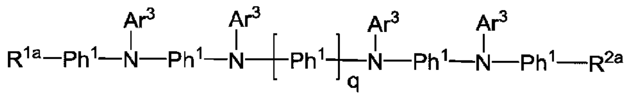 (1d)

wherein

$R^{1a}$ and $R^{2a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^3$ each, independently, represents a diarylaminophenyl group;
q represents 1 or 2; and
$Ph^1$ is the same as defined for formula (1c) above.

Specific examples of the halogen atom, the alkyl group having 1 to 20 carbon atoms, the alkenyl group having 2 to 20 carbon atoms, the alkynyl group having 2 to 20 carbon atoms, the aryl group having 6 to 20 carbon atoms, and the heteroaryl group having 2 to 20 carbon atoms include the same as those listed above.

Among these, $R^{1a}$ and $R^{2a}$ are preferably hydrogen atoms, fluorine atoms, cyano groups, alkyl groups having 1 to 20 carbon atoms which may be substituted with a halogen atom, aryl groups having 6 to 20 carbon atoms which may be substituted with a halogen atom, or heteroaryl groups having 2 to 20 carbon atoms which may be substituted with a halogen atom; more preferably, hydrogen atoms, fluorine atoms, cyano groups, alkyl groups having 1 to 10 carbon atoms which may be substituted with a halogen atom, or phenyl groups which may be substituted with a halogen atom; even more preferably, hydrogen atoms or fluorine atoms; and most preferably, hydrogen atoms.

Preferably, $Ar^3$ each, independently, represents a group represented by any of formulas (11) to (18) below.

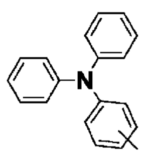 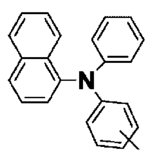 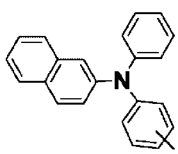 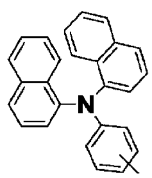 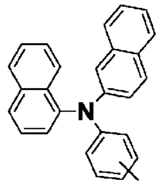

(I1) (I2) (I3) (I4) (I5)

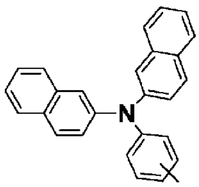
(I6)

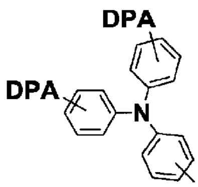
(I7)

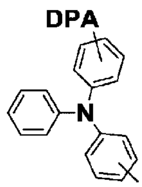
(I8)

wherein DPA represents a diphenylamino group.

More preferably, $Ar^3$ each, independently, represents a group represented by any of (I1') to (I8') below.

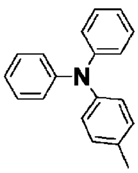
(I1')

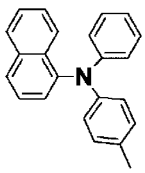
(I2')

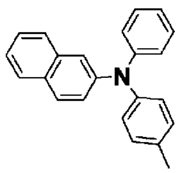
(I3')

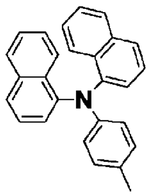
(I4')

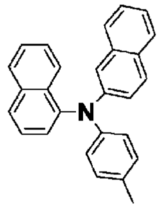
(I5')

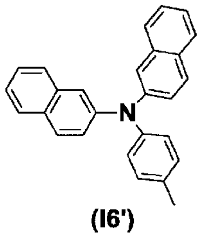
(I6')

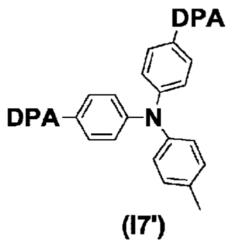
(I7')

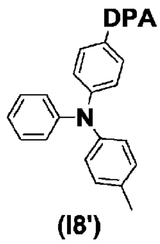
(I8')

wherein DPA represents a diphenylamino group.

- Oligoaniline compound (v)

The oligoaniline compound (v) that can be used as the oligoaniline compound (a) is an oligoaniline compound represented by formula (1e) below:

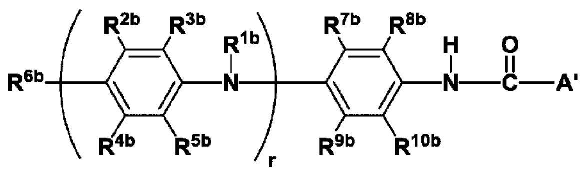
(1e)

wherein

$R^{1b}$ represents a hydrogen atom or an alkyl group having 1 to 20 carbon atoms optionally substituted with $Z^b$;
$Z^b$ represents a halogen atom, a nitro group, a cyano group, an aldehyde group, a hydroxyl group, a thiol group, a sulfonic acid group, a carboxyl group, an aryl group having 6 to 20 carbon atoms optionally substituted with $Z^{b'}$, or a heteroaryl group having 2 to 20 carbon atoms optionally substituted with $Z^{b'}$;
$Z^{b'}$ represents a halogen atom, a nitro group, a cyano group, an aldehyde group, a hydroxyl group, a thiol group, a sulfonic acid group or a carboxyl group;
$R^{2b}$ to $R^{10b}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
A' represents a hydrocarbon group having 1 to 40 carbon atoms, wherein at least one hydrogen atom is substituted with a fluorine atom and at least one other hydrogen atom is substituted with another atom or substituent; and

r is an integer from 1 to 20.

Specific examples of the halogen atom, the alkyl group having 1 to 20 carbon atoms, the alkenyl group having 2 to 20 carbon atoms, the alkynyl group having 2 to 20 carbon atoms, the aryl group having 6 to 20 carbon atoms, and the heteroaryl group having 2 to 20 carbon atoms include the same as those listed above.

Of these, considering the solubility of the oligoaniline compound in organic solvents, $R^{1b}$ is preferably a hydrogen atom or an alkyl group having 1 to 10 carbon atoms which may be substituted with $Z^b$; more preferably, a hydrogen atom or an alkyl group having 1 to 4 carbon atoms which may be substituted with $Z^b$; and most preferably, a hydrogen atom. In cases where there are a plurality of $R^{1b}$, they may each be the same or may be different.

Of these, considering the solubility of the oligoaniline compound in organic solvents, $R^{2b}$ to $R^{10b}$ are preferably hydrogen atoms, halogen atoms, nitro groups, cyano groups or alkyl groups having 1 to 10 carbon atoms which may be substituted with halogen atoms; and more preferably hydrogen atoms, halogen atoms or alkyl groups having 1 to 4 carbon atoms which may be substituted with halogen atoms. Considering the balance between the solubility in organic solvents of, and the charge transportability of, the oligoaniline compound, $R^{2b}$ to $R^{10b}$ are most preferably hydrogen atoms. In cases where there are a plurality of $R^{2b}$ to $R^{5b}$, they may each be the same or may be different.

Preferably, A' is:

a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms which may be substituted with a cyano group, a chlorine atom, a bromine atom, an iodine atom, a nitro group or a fluoroalkoxy group having 1 to 20 carbon atoms;
a fluoroaryl group having 6 to 20 carbon atoms which may be substituted with a cyano group, a chlorine atom, a bromine atom, an iodine atom, a nitro group, an alkyl group having 1 to 20 carbon atoms, a fluoroalkyl group having 1 to 20 carbon atoms or a fluoroalkoxy group having 1 to 20 carbon atoms;
an aryl group having 6 to 20 carbon atoms which is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms and which may also be substituted with a cyano group, a halogen atom or a fluoroalkoxy group having 1 to 20 carbon atoms;
a fluoroaralkyl group having 7 to 20 carbon atoms which may be substituted with a cyano group, a chlorine atom, a bromine atom, an iodine atom, a nitro group, a fluoroalkoxy group having 1 to 20 carbon atoms, a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms; or
an aralkyl group having 7 to 20 carbon atoms which is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms and which may also be substituted with a cyano group, a halogen atom or a fluoroalkoxy group having 1 to 20 carbon atoms.

The fluoroalkyl group is not particularly limited, provided that it is a linear or branched alkyl group in which at least one hydrogen atom on a carbon atom is substituted with a fluorine atom. Examples include fluoromethyl, difluoromethyl, trifluoromethyl, 1-fluoroethyl, 2-fluoroethyl, 1,2-difluoroethyl, 1,1-difluoroethyl, 2,2-difluoroethyl, 1,1,2-trifluoroethyl, 1,2,2-trifluoroethyl, 2,2,2-trifluoroethyl, 1,1,2,2-tetrafluoroethyl, 1,2,2,2-tetrafluoroethyl, 1,1,2,2,2-pentafluoroethyl, 1-fluoropropyl, 2-fluoropropyl, 3-fluoropropyl, 1,1-difluoropropyl, 1,2-difluoropropyl, 1,3-difluoropropyl, 2,2-difluoropropyl, 2,3-difluoropropyl, 3,3-difluoropropyl, 1,1,2-trifluoropropyl, 1,1,3-trifluoropropyl, 1,2,3-trifluoropropyl, 1,3,3-trifluoropropyl, 2,2,3-trifluoropropyl, 2,3,3-trifluoropropyl, 3,3,3-trifluoropropyl, 1,1,2,2-tetrafluoropropyl, 1,1,2,3-tetrafluoropropyl, 1,2,2,3-tetrafluoropropyl, 1,3,3,3 -tetrafluoropropyl, 2,2,3,3 -tetrafluoropropyl, 2,3,3,3 -tetrafluoropropyl, 1,1,2,2,3-pentafluoropropyl, 1,2,2,3,3-pentafluoropropyl, 1,1,3,3,3-pentafluoropropyl, 1,2,3,3,3-pentafluoropropyl, 2,2,3,3,3-pentafluoropropyl and heptafluoropropyl groups and the like.

The fluorocycloalkyl group is not particularly limited, provided that it is a cycloalkyl group in which at least one hydrogen atom on a carbon atom is substituted with a fluorine atom. Examples include 1-fluorocyclopropyl, 2-fluorocyclopropyl, 2,2-difluorocyclopropyl, 2,2,3,3-tetrafluorocyclopropyl, pentafluorocyclopropyl, 2,2-difluorocyclobutyl, 2,2,3,3-tetrafluorocyclobutyl, 2,2,3,3,4,4-hexafluorocyclobutyl, heptafluorocyclobutyl, 1-fluorocyclopentyl, 3-fluorocyclopentyl, 3,3-difluorocyclopentyl, 3,3,4,4-tetrafluorocyclopentyl, nonafluorocyclopentyl, 1-fluorocyclohexyl, 2-fluorocyclohexyl, 4-fluorocyclohexyl, 4,4-difluorocyclohexyl, 2,2,3,3-tetrafluorocyclohexyl, 2,3,4,5,6-pentafluorocyclohexyl and undecafluorocyclohexyl groups and the like.

The fluorobicycloalkyl group is not particularly limited, provided that it is a bicycloalkyl group in which at least one hydrogen atom on a carbon atom is substituted with a fluorine atom. Examples include 3-fluorobicyclo[1.1.0]butan-1-yl, 2,2,4,4-tetrafluorobicyclo[1.1.0]butan-1-yl, pentafluorobicyclo[1.1.0]butan-1-yl, 3-fluorobicyclo[1.1.1]pentan-1-yl,

2,2,4,4,5-pentafluorobicyclo[1.1.1]pentan-1-yl, 2,2,4,4,5,5-hexafluorobicyclo[1.1.1]pentan-1-yl, 5-fluorobicyclo[3.1.0]hexan-6-yl, 6-fluorobicyclo[3.1.0]hexan-6-yl, 6,6-difluorobicyclo[3.1.0]hexan-2-yl, 2,2,3,3,5,5,6,6-octafluorobicyclo[2.2.0]hexan-1-yl, 1-fluorobicyclo[2.2.1]heptan-2-yl, 3-fluorobicyclo[2.2.1]heptan-2-yl, 4-fluorobicyclo[2.2.1]heptan-1-yl, 5-fluorobicyclo[3.1.1]heptan-1-yl, 1,3,3,4,5,5,6,6,7,7-decafluorobicyclo[2.2.1]heptan-2-yl, undecafluorobicyclo[2.2.1]heptan-2-yl, 3-fluorobicyclo[2.2.2]octan-1-yl and 4-fluorobicyclo[2.2.2]octan-1-yl groups and the like.

The fluoroalkenyl group is not particularly limited, provided that it is an alkenyl group in which at least one hydrogen atom on a carbon atom is substituted with a fluorine atom. Examples include 1-fluoroethenyl, 2-fluoroethenyl, 1,2-difluoroethenyl, 1,2,2-trifluoroethenyl, 2,3,3-trifluoro-1-propenyl, 3,3,3-trifluoro-1-propenyl, 2,3,3,3-tetrafluoro-1-propenyl, pentafluoro-1-propenyl, 1-fluoro-2-propenyl, 1,1-difluoro-2-propenyl, 2,3-difluoro-2-propenyl, 3,3-difluoro-2-propenyl, 2,3,3-trifluoro-2-propenyl, 1,2,3,3-tetrafluoro-2-propenyl and pentafluoro-2-propenyl groups and the like.

The fluoroalkynyl group is not particularly limited, provided that it is an alkynyl group in which at least one hydrogen atom on a carbon atom is substituted with a fluorine atom. Examples include fluoroethynyl, 3-fluoro-1-propynyl, 3,3-difluoro-1-propynyl, 3,3,3-trifluoro-1-propynyl, 1-fluoro-2-propynyl and 1,1-difluoro-2-propynyl groups and the like.

The fluoroaryl group is not particularly limited, provided that it is an aryl group in which at least one hydrogen atom on a carbon atom is substituted with a fluorine atom. Examples include 2-fluorophenyl, 3-fluorophenyl, 4-fluorophenyl, 2,3-difluorophenyl, 2,4-difluorophenyl, 2,5-difluorophenyl, 2,6-difluorophenyl, 3,4-difluorophenyl, 3,5-difluorophenyl, 2,3,4-trifluorophenyl, 2,3,5-trifluorophenyl, 2,3,6-trifluorophenyl, 2,4,5-trifluorophenyl, 2,4,6-trifluorophenyl, 3,4,5-trifluorophenyl, 2,3,4,5-tetrafluorophenyl, 2,3,4,6-tetrafluorophenyl, 2,3,5,6-tetrafluorophenyl, pentafluorophenyl, 2-fluoro-1-naphthyl, 3-fluoro-1-naphthyl, 4-fluoro-1-naphthyl, 6-fluoro-1-naphthyl, 7-fluoro-1-naphthyl, 8-fluoro-1-naphthyl, 4,5-difluoro-1-naphthyl, 5,7-difluoro-1-naphthyl, 5,8-difluoro-1-naphthyl, 5,6,7,8-tetrafluoro-1-naphthyl, heptafluoro-1-naphthyl, 1-fluoro-2-naphthyl, 5-fluoro-2-naphthyl, 6-fluoro-2-naphthyl, 7-fluoro-2-naphthyl, 5,7-difluoro-2-naphthyl and heptafluoro-2-naphthyl groups and the like.

The fluoroaryl group is preferably a phenyl group which is substituted with three or more fluorine atoms and which may be substituted with a cyano group, a chlorine atom, a bromine atom, an iodine atom, a nitro group, an alkyl group having 1 to 20 carbon atoms, a fluoroalkyl group having 1 to 20 carbon atoms or a fluoroalkoxy group having 1 to 20 carbon atoms, considering the balance between the solubility of the oligoaniline compound in organic solvents, the charge transportability of the oligoaniline compound, the availability of starting materials for the oligoaniline compound and the like.

The fluoroalkoxy group is not particularly limited, provided that it is an alkoxy group in which at least one hydrogen atom on a carbon atom is substituted with a fluorine atom. Examples include fluoromethoxy, difluoromethoxy, trifluoromethoxy, 1-fluoroethoxy, 2-fluoroethoxy, 1,2-difluoroethoxy, 1,1-difluoroethoxy, 2,2-difluoroethoxy, 1,1,2-trifluoroethoxy, 1,2,2-trifluoroethoxy, 2,2,2-trifluoroethoxy, 1,1,2,2-tetrafluoroethoxy, 1,2,2,2-tetrafluoroethoxy, 1,1,2,2,2-pentafluoroethoxy, 1-fluoropropoxy, 2-fluoropropoxy, 3-fluoropropoxy, 1,1-difluoropropoxy, 1,2-difluoropropoxy, 1,3-difluoropropoxy, 2,2-difluoropropoxy, 2,3-difluoropropoxy, 3,3 -difluoropropoxy, 1,1,2-trifluoropropoxy, 1,1,3 -trifluoropropoxy, 1,2,3-trifluoropropoxy, 1,3,3-trifluoropoxy, 2,2,3-trifluoropropoxy, 2,3,3-trifluoropropoxy, 3,3,3-trifluoropropoxy, 1,1,2,2-tetrafluoropropoxy, 1,1,2,3-tetrafluoropropoxy, 1,2,2,3-tetrafluoropropoxy, 1,3,3,3-tetrafluoropropoxy, 2,2,3,3-tetrafluoropropoxy, 2,3,3,3-tetrafluoropropoxy, 1,1,2,2,3-pentafluoropropoxy, 1,2,2,3,3-pentafluoropropoxy, 1,1,3,3,3-pentafluoropropoxy, 1,2,3,3,3-pentafluoropropoxy, 2,2,3,3,3-pentafluoropropoxy and heptafluoropropoxy groups and the like.

The aryl group having 6 to 20 carbon atoms which is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms and which may also be substituted with a cyano group, a halogen atom or a fluoroalkoxy group having 1 to 20 carbon atoms (which aryl group is also referred to below, for the sake of convenience, as the "substituted aryl group") is not particularly limited, provided that it is an aryl group in which at least one hydrogen atom on a carbon atom is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms. Examples include 2-(trifluoromethyl)phenyl, 3-(trifluoromethyl)phenyl, 4-(trifluoromethyl)phenyl, 4-ethoxy-3-(trifluoromethyl)phenyl, 3-fluoro-4-(trifluoromethyl)phenyl, 4-fluoro-3-(trifluoromethyl)phenyl, 4-fluoro-2-(trifluoromethyl)phenyl, 2-fluoro-5-(trifluoromethyl)phenyl, 3-fluoro-5-(trifluoromethyl)phenyl, 3,5-di(trifluoromethyl)phenyl, 2,4,6-tri(trifluoromethyl)phenyl, 4-(pentafluoroethyl)phenyl, 4-(3,3,3-trifluoropropyl)phenyl, 2,3,5,6-tetrafluoro-4-(trifluoromethyl)phenyl, 4-(perfluorovinyl)phenyl, 4-(perfluoropropenyl)phenyl and 4-(perfluorobutenyl)phenyl groups and the like.

Considering the balance between the solubility of the oligoaniline compound in organic solvents, the charge transportability of the oligoaniline compound, the availability of starting materials for the oligoaniline compound and the like, the substituted aryl group is preferably a phenyl group which is substituted with a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms and which may also be substituted with a cyano group, a halogen atom or a fluoroalkoxy group having 1 to 20 carbon atoms (which phenyl group is also referred to below, for the sake of convenience, as the "substituted phenyl group"); more preferably, a phenyl group substituted with from one

to three trifluoromethyl groups; and even more preferably a p-trifluoromethylphenyl group.

The fluoroaralkyl group is not particularly limited, provided it is an aralkyl group in which at least one hydrogen atom on a carbon atom is substituted with a fluorine atom. Examples include 2-fluorobenzyl, 3-fluorobenzyl, 4-fluorobenzyl, 2,3-difluorobenzyl, 2,4-difluorobenzyl, 2,5-difluorobenzyl, 2,6-difluorobenzyl, 3,4-difluorobenzyl, 3,5-difluorobenzyl, 2,3,4-trifluorobenzyl, 2,3,5-trifluorobenzyl, 2,3,6-trifluorobenzyl, 2,4,5-trifluorobenzyl, 2,4,6-trifluorobenzyl, 2,3,4,5-tetrafluorobenzyl, 2,3,4,6-tetrafluorobenzyl, 2,3,5,6-tetraflurobenzyl and 2,3,4,5,6-pentafluorobenzyl groups and the like.

The aralkyl group having 7 to 20 carbon atoms which is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms and which may also be substituted with a cyano group, a halogen atom or a fluoroalkoxy group having 1 to 20 carbon atoms is not particularly limited, provided it is an aralkyl group in which at least one hydrogen atom on a carbon atom is substituted with a fluoroalkyl group having 1 to 20 carbon atoms, a fluorocycloalkyl group having 3 to 20 carbon atoms, a fluorobicycloalkyl group having 4 to 20 carbon atoms, a fluoroalkenyl group having 2 to 20 carbon atoms or a fluoroalkynyl group having 2 to 20 carbon atoms. Examples include 2-trifluoromethylbenzyl, 3-trifluoromethylbenzyl, 4-trifluoromethylbenzyl, 2,4-di(trifluoromethyl)benzyl, 2,5-di(trifluoromethyl)benzyl, 2,6-di(trifluoromethyl)benzyl, 3,5-di(trifluoromethyl)benzyl and 2,4,6-tri(trifluoromethyl)benzyl groups and the like.

Of these, A' is preferably the above fluoroalkyl group having 1 to 20 carbon atoms which may be substituted, the above fluoroaryl group having 6 to 20 carbon atoms which may be substituted or the above substituted aryl group; more preferably, the above fluoroaryl group having 6 to 20 carbon atoms which may be substituted or the above substituted aryl group; even more preferably, the above fluorophenyl group which may be substituted or the above substituted phenyl group; and still more preferably, the above trifluorophenyl group which may be substituted, the above tetrafluorophenyl group which may be substituted, the above pentafluorophenyl group which may be substituted or a phenyl group substituted with from one to three trifluoromethyl groups.

Also, r is an integer from 1 to 20. However, from the perspective of the solubility of the oligoaniline compound in solvents, r is preferably 10 or less, more preferably 8 or less, even more preferably 5 or less, and still more preferably 4 or less. From the perspective of increasing the charge transportability of the oligoaniline compound, r is preferably 2 or greater, and more preferably 3 or greater. Considering the balance between solubility and charge transportability, r is most preferably 3.

Specific examples of oligoaniline compounds suitable for the present invention include, but are not limited to, the following.

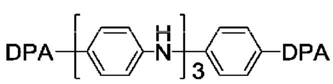
(A1)

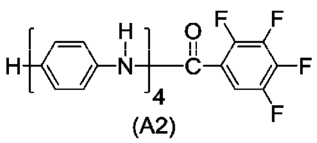
(A2)

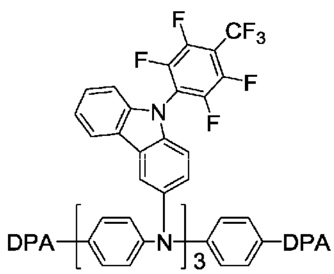
(A3)

wherein DPA represents a diphenylamino group.

< Sulfonic acid ester compound (b) >

The sulfonic acid ester compound (b) contained in the charge transporting varnish of the present invention is a sulfonic acid ester compound represented by formula (2) below:

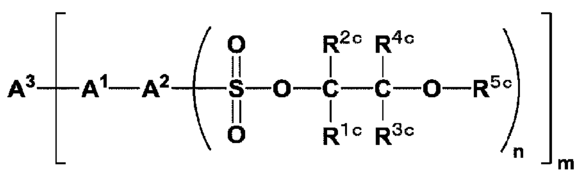 **(2)**

wherein

$R^{1c}$ to $R^{4c}$ each, independently, represent a hydrogen atom or a linear or branched alkyl group having 1 to 6 carbon atoms,
$R^{5c}$ represents an optionally substituted monovalent hydrocarbon group having 2 to 20 carbon atoms,
$A^1$ represents -O- or -S-,
$A^2$ represents an (n+1)-valent group derived from naphthalene or anthracene,
$A^3$ represents an m-valent group derived from perfluorobiphenyl,
m represents an integer satisfying $2 \leq m \leq 4$, and
n represents an integer satisfying $1 \leq n \leq 4$.

This compound functions as a precursor of the electron accepting substance in the charge transporting varnish of the present invention. An electron accepting substance is a substance that is used to enhance charge transportability and uniformity in film formation, and is synonymous with an electron accepting dopant.

Examples of the linear or branched alkyl group include, but are not limited to, a methyl group, an ethyl group, an n-propyl group, an isopropyl group, an n-butyl group, an isobutyl group, a tert-butyl group, an n-hexyl group and the like. Of these, an alkyl group having 1 to 3 carbon atoms is preferred.

Examples of the monovalent hydrocarbon group having 2 to 20 carbon atoms include alkyl groups such as an ethyl group, an n-propyl group, an isopropyl group, an n-butyl group, an isobutyl group and a tert-butyl group, aryl groups such as a phenyl group, a naphthyl group and a phenanthryl group, and the like.

It is preferable for $R^{1c}$ or $R^{3c}$ to be a linear alkyl group having 1 to 3 carbon atoms and for the remainder of $R^{1c}$ to $R^{4c}$ to be hydrogen atoms. It is more preferable for $R^{1c}$ to be a linear alkyl group having 1 to 3 carbon atoms, and for $R^{2c}$ to $R^{4c}$ to be hydrogen atoms. The linear alkyl group having 1 to 3 carbon atoms is preferably a methyl group. $R^{5c}$ is preferably a linear alkyl group having 2 to 4 carbon atoms or a phenyl group.

$A^1$ is preferably -O-. $A^2$ is preferably a group derived from naphthalene.

m is preferably 2. n is preferably 1 or 2.

Because the sulfonic acid ester compound (b) exhibits high solubility in a broad range of solvents, including low polarity solvents, coatability can be improved by using a variety of solvents to adjust the properties of the solution. Therefore, it is preferable that a coating film be formed by applying a charge transporting varnish comprising the sulfonic acid ester, and a sulfonic acid be then generated from the sulfonic acid ester on drying or baking the coating film. Considering that it is preferable for the sulfonic acid ester to be stable at room temperature and for the sulfonic acid to be generated at or below the baking temperature, the temperature at which the sulfonic acid is generated from the sulfonic acid ester is preferably 40 to 260°C. Further, taking into account the high stability of the sulfonic acid ester in the varnish and the ease of reaction during baking, the temperature is preferably from 80 to 230°C, and more preferably from 120 to 180°C.

The sulfonic acid ester compound (b) can be rendered into a charge transporting varnish by dissolution or dispersion, together with the oligoaniline compound (a), which serves as the main component of the charge transport mechanism, in an organic solvent.

The sulfonic acid ester compound (b) (formula (2)) can be synthesized by, for example, reacting a sulfonic acid salt compound represented by formula (2") with a halogenating agent to synthesize a sulfonyl halide compound represented by formula (2') below (hereafter also referred to as "Step 1"), and then allowing this sulfonyl halide compound to react with a glycol ether represented by formula (3) (hereafter also referred to as "Step 2"), as shown in Scheme A below:

[Scheme A]

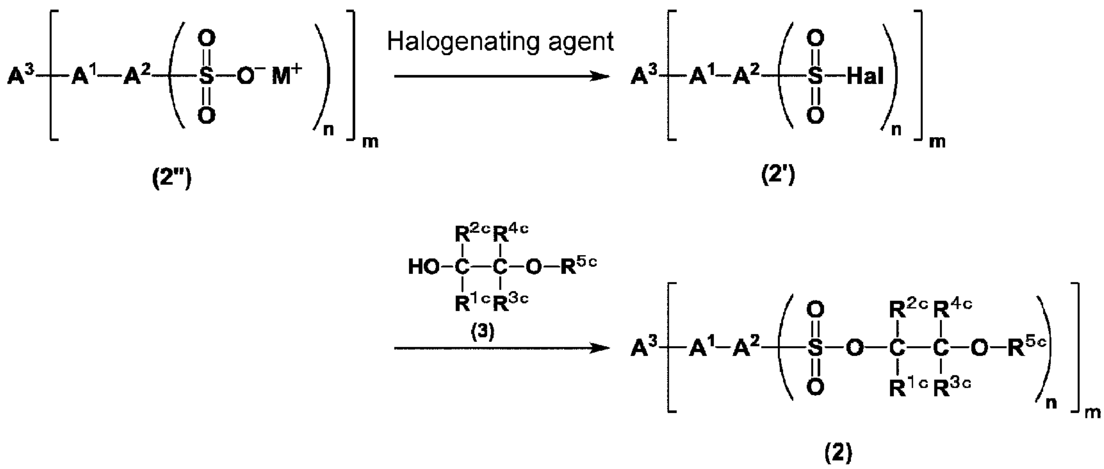


wherein $A^1$ to $A^3$, $R^{1c}$ to $R^{5c}$, m and n are as defined above; $M^+$ represents a monovalent cation such as a sodium ion, a potassium ion, a pyridinium ion or a quaternary ammonium ion; and Hal represents a halogen atom such as a chlorine atom or a bromine atom.

The sulfonic acid salt compound represented by formula (2") can be synthesized by methods known in the art.

Examples of the halogenating agent used in Step 1 include halogenating agents such as thionyl chloride, oxalyl chloride, phosphorus oxychloride and phosphorus(V) chloride; and thionyl chloride is preferred. The amount of halogenating agent used is not limited, as long as it is at least one mole per mole of the sulfonic acid salt compound, but it is preferably used in an amount from 2 to 10 times the amount by mass of the sulfonic acid salt compound.

The reaction solvent used in Step 1 is preferably a solvent that does not react with the halogenating agent, examples of which include chloroform, dichloroethane, carbon tetrachloride, hexane and heptane, although it is preferable to use no solvent (a liquid halogenating agent is used as a solvent). When the reaction is carried out in the absence of a solvent, the halogenating agent is preferably used in an amount equal to or more than the amount at which the system becomes a homogeneous solution when the reaction is completed. The reaction temperature may be set to from about 0°C to about 150°C, although the reaction temperature is preferably from 20 to 100°C and at or below the boiling point of the halogenating agent used. Following the completion of the reaction, the crude product obtained by vacuum concentration or the like is typically used in the next step.

Preferred examples of the glycol ether represented by formula (3) include propylene glycol monoethyl ether, propylene glycol monopropyl ether, propylene glycol monobutyl ether, propylene glycol monophenyl ether, ethylene glycol monobutyl ether and ethylene glycol monohexyl ether.

In Step 2, a base may be concomitantly used. Examples of bases that may be used include sodium hydride, pyridine, triethylamine and diisopropylethylamine; sodium hydride, pyridine and triethylamine are preferred. The base is preferably used in an amount that ranges from one mole per mole of the sulfonyl halide compound (2') up to a solvent amount.

Various organic solvents may be used as the reaction solvent in Step 2, but tetrahydrofuran, dichloroethane, chloroform and pyridine are preferred. The reaction temperature, although not particularly limited, is preferably from 0 to 80°C. Following the completion of the reaction, the sulfonic acid ester compound can be obtained in pure form by work-up and purification using customary methods such as vacuum concentration, liquid/liquid extraction, water rinsing, reprecipitation, recrystallization and chromatography. Further, the pure sulfonic acid ester compound thus obtained can optionally be rendered into a high-purity sulfonic acid compound by subjecting it to heat treatment or the like.

Alternatively, as shown in Scheme B below, the sulfonic acid ester compound represented by formula (2) can also be synthesized from a sulfonic acid compound represented by formula (2"'). In the scheme shown below, the halogenating agent, the glycol ether represented by formula (3), the reaction solvents and other ingredients used in the first-stage and second-stage reactions may be the same as those used in Steps 1 and 2 of Scheme A.

[Scheme B]

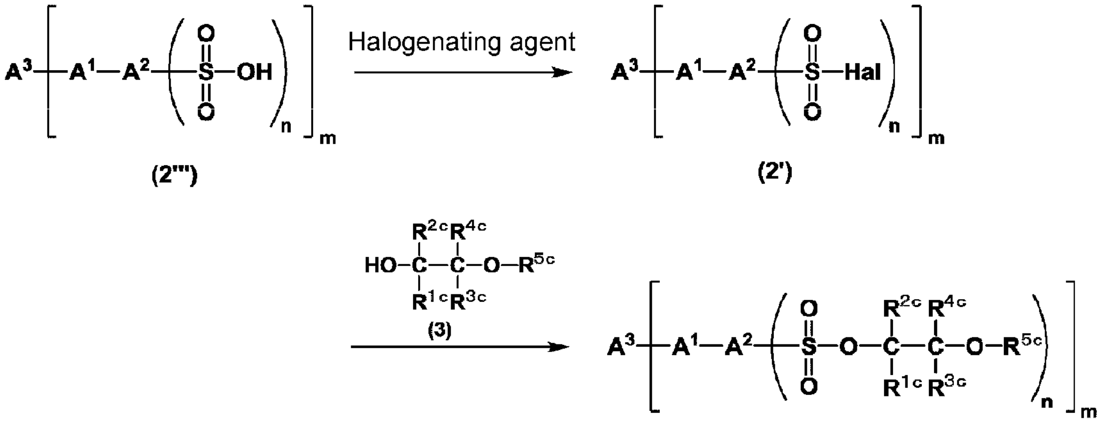

(2''')
(2')
(3)
(2)

wherein $A^1$ to $A^3$, $R^{1c}$ to $R^{5c}$, m, n and Hal are as defined above.

The sulfonic acid compound represented by formula (2''') may be synthesized, for example, according to the method described in WO 2006/025342.

Specific examples of sulfonic acid ester compounds suitable for the present invention include, but are not limited to, the following.

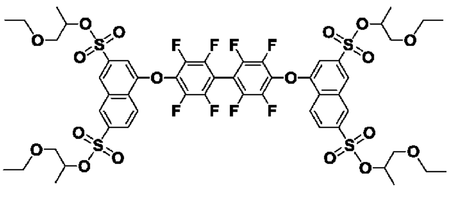
(S1)

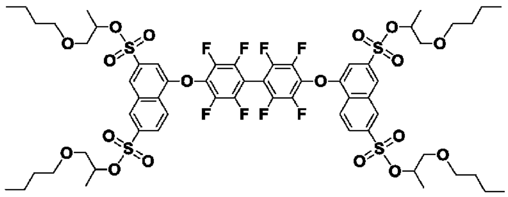
(S2)

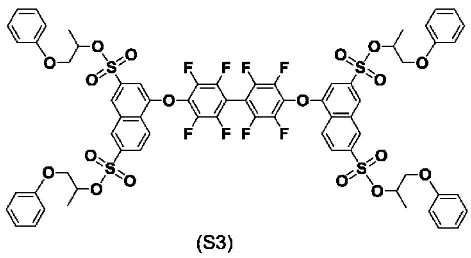
(S3)

< Metal oxide nanoparticles (c) >

The charge transporting varnish of the present invention comprises one or more metal oxide nanoparticles (c). A "nanoparticle" refers to a fine particle having a primary particle average diameter on the order of nanometers (typically, 500 nm or less). A "metal oxide nanoparticle" refers to an oxide of a metal formed into a nanoparticle.

Metals for the metal oxide nanoparticles (c) include metalloids in addition to metals in the usual sense. Metals in the usual sense may be used alone or in combination of two or more, and those used preferably include, but are not limited to, at least one selected from the group consisting of tin (Sn), titanium (Ti), aluminum (Al), zirconium (Zr), zinc (Zn), niobium (Nb), tantalum (Ta), and W (tungsten).

By contrast, a "metalloid" refers to a chemical element that has chemical and/or physical properties in between those of metals and nonmetals. Although no universal definition of metalloids has been established, in the present invention, the term "metalloid" refers to the following six elements: boron (B), silicon (Si), germanium (Ge), arsenic (As), antimony (Sb), and tellurium (Te). These metalloids may be used alone or in combination of two or more, or may be used in combination with a metal in the usual sense.

The metal oxide nanoparticles (c) preferably comprise an oxide of at least one metal selected from the group consisting of boron (B), silicon (Si), germanium (Ge), arsenic (As), antimony (Sb), tellurium (Te), tin (Sn), titanium (Ti), aluminum (Al), zirconium (Zr), zinc (Zn), niobium (Nb), tantalum (Ta), and W (tungsten). When the metal is a combination of two or more metals, the metal oxide may be a mixture of oxides of individual single metals or may be a composite oxide comprising a plurality of metals. Specific examples of metal oxides include, but are not limited to $B_2O_3$, $B_2O$, $SiO_2$, SiO, $GeO_2$, GeO, $As_2O_4$, $As_2O_3$, $As_2O_5$, $Sb_2O_3$, $Sb_2O_5$, $TeO_2$, $SnO_2$, $ZrO_2$, $Al_2O_3$, ZnO and the like.

In an embodiment, the metal oxide nanoparticles (c) comprise $B_2O_3$, $B_2O$, $SiO_2$, SiO, $GeO_2$, GeO, $As_2O_4$, $As_2O_3$, $As_2O_5$, $SnO_2$, SnO, $Sb_2O_3$, $TeO_2$, or mixtures thereof. In another embodiment, the metal oxide nanoparticles (c) comprise $SiO_2$.

The metal oxide nanoparticles have a primary particle average diameter typically in the range of 1 nm or greater and 500 nm or less, preferably 1 nm or greater and 250 nm or less, more preferably about 1 nm or greater and about 100 nm or less, still more preferably 1 nm or greater and 50 nm or less, particularly preferably about 2 nm or greater and about 30 nm or less, and most preferably 3 nm or greater and 25 nm or less. Examples of methods for measuring the average particle diameter of primary particles include, for example, a method using a transmission electron microscope (TEM), a method of calculation based on the specific surface area obtained by the BET method, and the like.

Various methods are known in the art for measuring the average particle diameter that involve the use of TEM, and one example thereof is a method based on circle equivalent diameters. In this method, a projected image of particles obtained by using a TEM (e.g., the transmission electron microscope HT7700, manufactured by Hitachi High Technologies Corporation) is processed by image processing software to determine the circle equivalent diameters of the individual particles, and the average particle diameter is calculated as the number average of these circle equivalent diameters. The circle equivalent diameter is also called the Haywood diameter, and is the diameter of a circle having the same area as that of a projected image of a particle. In this method, projection images are typically processed using the image processing software provided with a TEM and created by the TEM manufacturer.

The metal oxide nanoparticles (c) may comprise one or more organic capping groups. Such organic capping groups may be reactive or non-reactive. Examples of reactive organic capping groups include organic capping groups that can be cross-linked by ultraviolet light or a radical initiator. In an embodiment, the metal oxide nanoparticles (c) comprise one or more organic capping groups.

The metal oxide nanoparticles can be produced by methods known in the art, but are also available as commercial products. Commercially available metal oxide nanoparticles are typically in the form of a dispersion. Preferably, a non-aqueous dispersion of metal oxide nanoparticles that is commercially available is used. Examples of suitable commercially available metal oxide nanoparticles include ORGANOSILICASOL™ products, manufactured by Nissan Chemical Industries, Ltd., which are non-aqueous dispersions in which $SiO_2$ nanoparticles are dispersed in various solvents, such as, for example, methanol, methyl ethyl ketone, methyl isobutyl ketone, N,N-dimethylacetamide, ethylene glycol, isopropanol, methanol, ethylene glycol monopropyl ether, cyclohexanone, ethyl acetate, toluene, and propylene glycol monomethyl ether acetate.

The amount of the metal oxide nanoparticles (c) in the charge transporting varnish of the present invention is expressed as a weight percentage relative to the combined weight of the metal oxide nanoparticles (c) and the oligoaniline compound (a) (including doped and undoped forms). The amount of the metal oxide nanoparticles (c) is typically from about 1 wt. % to about 98 wt. %, preferably from about 2 wt. to about 95 wt. %, more preferably from about 5 wt. % to about 90 wt. %, still more preferably about 10 wt. % to about 90 wt. %, relative to the combined weight of the metal oxide nanoparticles (c) and the oligoaniline compound (a). In an embodiment, the amount of the metal oxide nanoparticles (c) is from about 20 wt. % to about 98 wt. %, preferably from about 25 wt. to about 95 wt. %, relative to the combined weight of the metal oxide nanoparticles (c) and the oligoaniline compound (a).

< Organic solvent (d) >

The charge transporting varnish of the present disclosure may be non-aqueous or may comprise water, but is preferably non-aqueous from the perspective of process compatibility in inkjet coating and the storage stability of the varnish. As used herein, "non-aqueous" means that the total amount of water in the charge transporting varnish of the present disclosure is from 0 to 2 wt. %, with respect to the total amount of charge transporting varnish. Typically, the total amount of water in the charge transporting varnish is from 0 to 1 wt. %, more typically from 0 to 0.5 wt. %, with respect to the total amount of charge transporting varnish. In an embodiment, the charge transporting varnish of the present disclosure is substantially free of water.

Examples of the organic solvent used for the charge transporting varnish include, for example, N,N-dimethylformamide (153°C), N,N-dimethylacetamide (165°C), N-methylpyrrolidone (202°C), 1,3-dimethyl-2-imidazolidinone (225°C), dimethylsulfoxide (189°C), N-cyclohexyl-2-pyrrolidinone (284°C), aromatic hydrocarbons [such as benzene (80°C), toluene (111°C), ethylbenzene (136°C), p-xylene (138°C), o-xylene (138°C), and styrene (145°C)], ketones [such as acetone (56°C), methyl ethyl ketone (80°C), methyl isopropyl ketone (94°C), diethyl ketone (102°C), methyl isobutyl

ketone (117°C), methyl n-butyl ketone (127°C), cyclohexanone (155°C), and ethyl n-amyl ketone (167°C)], esters [such as ethyl acetate (77°C), isopropyl acetate (85°C), n-propyl acetate (101°C), isobutyl acetate (116°C), n-butyl acetate (125°C), n-amyl acetate (142°C), methyl caproate (151°C), 2-methylpentyl acetate (162°C), and n-butyl lactate (186°C)], glycol esters and glycol ethers [such as ethylene glycol dimethyl ether (85°C), propylene glycol monomethyl ether (119°C), ethylene glycol monomethyl ether (124°C), propylene glycol monoethyl ether (132°C), ethylene glycol monoethyl ether (136°C), ethylene glycol monoisopropyl ether (144°C), ethylene glycol methyl ether acetate (145°C), propylene glycol monomethyl ether acetate (146°C), ethylene glycol ethyl ether acetate (156°C), diethylene glycol dimethyl ether (162°C), propylene glycol monobutyl ether (170°C), ethylene glycol monobutyl ether (171°C), diethylene glycol diethyl ether (188°C), dipropylene glycol monomethyl ether (189°C), diethylene glycol monomethyl ether (194°C), dipropylene glycol monoethyl ether (198°C), diethylene glycol monoethyl ether (202°C), triethylene glycol dimethyl ether (216°C), diethylene glycol monoethyl ether acetate (217°C), and diethylene glycol (244°C)], alcohols [such as methanol (65°C), ethanol (78°C), isopropanol (82°C), tert-butanol (83°C), allyl alcohol (97°C), n-propanol (97°C), 2-methyl-2-butanol (102°C), isobutanol (108°C), n-butanol (117°C), 2-methyl-1-butanol (130°C), 1-pentanol (137°C), 2-methyl-1-pentanol (148°C), 2-ethyl hexanol (185°C), 1-octanol (196°C), ethylene glycol (197°C), hexylene glycol (198°C), trimethylene glycol (214°C), 1-methoxy-2-butanol (135°C), cyclohexanol (161°C), diacetone alcohol (166°C), furfuryl alcohol (170°C), tetrahydrofurfuryl alcohol (178°C), propylene glycol (187°C), benzyl alcohol (205°C), and 1,3-butanediol (208°C)], phenols [such as phenol (182°C) and m-cresol (202°C)], and ethers, carboxylic acids and derivatives thereof [such as isopropyl ether (68°C), 1,4-dioxane (101°C), anisole (154°C), acetic acid (117°C), and γ-butyrolactone (204°C)].

These organic solvents may be used alone or in combination with two or more.

In the present invention, it is possible to use a high-solvency solvent that readily dissolves the oligoaniline compound (a) and the sulfonic acid ester compound (b). Examples of such high-solvency solvents include N,N-dimethylformamide, N,N-dimethylacetamide, N-methylpyrrolidone, 1,3-dimethyl-2-imidazolidinone, diethylene glycol, dimethylsulfoxide, dimethyl isobutyramide and the like. These solvents may be used alone or in combination with two or more. The amount used of these solvents may be 5 to 100 wt. % relative to the amount of all the solvents used for the varnish.

Further, each constituent of the charge transporting vanish is preferably completely dissolved or uniformly dispersed in the solvent above.

Also, the charge transporting varnish of the present invention preferably comprises at least one high-viscosity organic solvent having a viscosity at 20°C of 10 to 200 mPa·s, particularly 50 to 150 mPa·s, and a boiling point, under normal pressure, of 50 to 300°C, particularly 150 to 250°C.

Examples of high-viscosity organic solvents include, for example, cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol, and hexylene glycol.

The ratio at which the high-viscosity organic solvent is added relative to all the solvents used for the varnish of the present invention is preferably within a range that does not result in solids precipitation/deposition. The preferable ratio of addition is 5 to 80 wt. % so long as no solids are precipitated/deposited.

In addition, other solvents capable of imparting flatness to the film at the time of baking may be added in a ratio of 1 to 90 wt. %, preferably 1 to 50 wt. %, relative to all the solvents used for the varnish, in order to improve wettability against substrates and control the surface tension, polarity, and boiling point of the solvent.

Examples of such solvents include butyl cellosolve, diethylene glycol dimethyl ether, diethylene glycol diethyl ether, triethylene glycol dimethyl ether, dipropylene glycol monomethyl ether, ethylcarbitol, diacetone alcohol, γ-butyrolactone, and ethyl lactate.

Specific examples of combinations of these solvents include, but are not limited to, for example, the following.

Examples of combinations of two solvents: 1,3-dimethyl-2-imidazolidinone and cyclohexanol; tetraethylene glycol and diethylene glycol monoethyl ether acetate; dipropylene glycol and diethylene glycol monoethyl ether acetate; triethylene glycol monomethyl ether and diethylene glycol isopropyl ether; diethylene glycol and diethylene glycol monoethyl ether acetate; diethylene glycol and triethylene glycol dimethyl ether; 3-phenoxytoluene and triethylene glycol dimethyl ether; 1,3-dimethyl-2-imidazolidinone and hexylene glycol; 1,3-dimethyl-2-imidazolidinone and diethylene glycol monoethyl ether acetate; N,N-dimethylacetamide and cyclohexanol; N,N-dimethylacetamide and diethylene glycol dimethyl ether; 1,3-dimethyl-2-imidazolidinone and dipropylene glycol monopropyl ether; N,N-dimethylacetamide and n-hexylene acetate; 1,3-dimethyl-2-imidazolidinone and diethylene glycol monoethyl ether acetate; 1,3-dimethyl-2-imidazolidinone and triethylene glycol dimethyl ether; 3-phenoxytoluene and tetralin; diethylene glycol and triethylene glycol dimethyl ether; 3-phenoxytoluene and triethylene glycol dimethyl ether; 1,3-dimethyl-2-imidazolidinone and 2,3-butanediol; and diethylene glycol monomethyl ether and propylene glycol monomethyl ether;

Examples of combinations of three solvents: 1,3-dimethyl-2-imidazolidinone, cyclohexanol and propylene glycol; 1,3-dimethyl-2-imidazolidinone, cyclohexanol and propylene glycol; 1,3-dimethyl-2-imidazolidinone, 2,3-butanediol and diethylene glycol monoethyl ether acetate; 1,3-dimethyl-2-imidazolidinone, cyclohexanol and 2,3-butanediol; 1,3-dimethyl-2-imidazolidinone, cyclohexanol and 2,3-butanediol; 1,3-dimethyl-2-imidazolidinone, 2,3-butanediol and dipropylene glycol monomethyl ether; 1,3-dimethyl-2-imidazolidinone, 2,3-butanediol and propylene glycol monomethylether; 1,3-dimethyl-2-imidazolidinone, triethylene glycol monomethyl ether and hexylene glycol; 1,3-dimethyl-2-imidazolidinone, 2,3-butanediol and dipropylene glycol monomethyl ether; 1,3-dimethyl-2-imidazolidinone, hexylene glycol and dipropylene glycol monomethyl ether; 1,3-dimethyl-2-imidazolidinone, diethylene glycol and diethylene glycol monomethyl ether; tetraethylene glycol, triethylene glycol monomethyl ether, and triethylene glycol dimethyl ether; tetraethylene glycol, triethylene glycol monomethyl ether and diethylene glycol monomethyl ether; diethylene glycol, triethylene glycol monomethyl ether and diethylene glycol monomethyl ether; triethylene glycol monomethyl ether, triethylene glycol dimethyl ether and 3-phenoxybenzyl alcohol; diethylene glycol, triethylene glycol dimethyl ether and 2-benzyloxyethanol; ethylene glycol, triethylene glycol dimethyl ether and 2-benzyloxyethanol; 1,3-dimethyl-2-imidazolidinone, 2-benzyloxyethanol and triethylene glycol dimethyl ether; diethylene glycol, ethylene glycol, triethylene glycol monomethyl ether and 2-benzyloxyethanol; 1,3-dimethyl-2-imidazolidinone, 2,3-butanediol and diethylene glycol monoethyl ether acetate; 1,3-dimethyl-2-imidazolidinone, hexylene glycol and diethylene glycol monoethyl ether acetate; 1,3-dimethyl-2-imidazolidinone, triethylene glycol monomethyl ether and dipropylene glycol monomethyl ether; 1,3-dimethyl-2-imidazolidinone, diethylene glycol monomethyl ether and cyclohexanol; 1,3-dimethyl-2-imidazolidinone, diethylene glycol, 2,3-butanediol and triethylene glycol dimethyl ether; diethylene glycol, triethylene glycol dimethyl ether and 2,3-butanediol; diethylene glycol, dipropylene glycol monomethyl ether and diethylene glycol monomethyl ether; diethylene glycol, dipropylene glycol monomethyl ether and 1,3-dimethyl-2-imidazolidinone; diethylene glycol, 2-benzyloxyethanol and dipropylene glycol monomethyl ether; 1,3-dimethyl-2-imidazolidinone, diethylene glycol and triethylene glycol monomethyl ether; 1,3-dimethyl-2-imidazolidinone, diethylene glycol and triethylene glycol monomethyl ether; 1,3-dimethyl-2-imidazolidinone, diethylene glycol and dipropylene glycol monomethyl ether; 1,3-dimethyl-2-imidazolidinone, dipropylene glycol and tripropylene glycol monomethyl ether; N,N-dimethylacetamide, cyclohexanol and diethylene glycol dimethyl ether; 1,3-dimethyl-2-imidazolidinone, dipropylene glycol monopropyl ether and diethylene glycol monoethyl ether acetate; 1,3-dimethyl-2-imidazolidinone, dipropylene glycol and dipropylene glycol monomethyl ether; 1,3-dimethyl-2-imidazolidinone, 2,3-butanediol and diethylene glycol monoethyl ether acetate; 1,3-dimethyl-2-imidazolidinone, 2-phenoxyethanol and 2,3-butanediol; 1,3-dimethyl-2-imidazolidinone, 1,3-butanediol and diethylene glycol monoethyl ether acetate; 1,3-dimethyl-2-imidazolidinone, methyl benzoate and 1,3-butanediol; 1,3-dimethyl-2-imidazolidinone, tetraethylene glycol and triethylene glycol monomethyl ether; 1,3-dimethyl-2-imidazolidinone, hexylene glycol and dipropylene glycol; 1,3-dimethyl-2-imidazolidinone, diethylene glycol monomethyl ether and diethylene glycol; 1,3-dimethyl-2-imidazolidinone, hexylene glycol and triethylene glycol dimethyl ether; 1,3-dimethyl-2-imidazolidinone, cyclohexanol, and diethylene glycol monoethyl ether acetate; 1,3-dimethyl-2-imidazolidinone, cyclohexanol, and diethylene glycol monobutyl ether acetate; tetraethylene glycol, triethylene glycol monomethyl ether and hexylene glycol; tetraethylene glycol, triethylene glycol monomethyl ether and diethylene glycol monoethyl ether acetate; dipropylene glycol, diethylene glycol monoethyl ether acetate and 1,3-dimethyl-2-imidazolidinone; 1,3-dimethyl-2-imidazolidinone, dipropylene glycol, and diethylene glycol monoethyl ether acetate; 1,3-dimethyl-2-imidazolidinone, triethylene glycol dimethyl ether and diethylene glycol; 1,3-dimethyl-2-imidazolidinone, 2,3-butanediol, and dipropylene glycol monomethyl ether; 1,3-dimethyl-2-imidazolidinone, 2,3-butanediol and dipropylene glycol monomethyl ether; and 1,3-dimethyl-2-imidazolidinone, diethylene glycol monomethyl ether and propylene glycol monomethyl ether; and

Examples of combinations of four solvents: diethylene glycol, triethylene glycol monomethyl ether, diethylene glycol monomethyl ether and dipropylene glycol monomethyl ether; diethylene glycol, triethylene glycol monomethyl ether, diethylene glycol monomethyl ether and diethylene glycol isopropyl ether; 1,3-dimethyl-2-imidazolidinone, triethylene glycol monomethyl ether, 2,3-butanediol, and diethylene glycol monoethyl ether acetate; 1,3-dimethyl-2-imidazolidinone, diethylene glycol, diethylene glycol monophenyl ether, and diethylene glycol monomethyl ether; 1,3-dimethyl-2-imidazolidinone, tetraethylene glycol, triethylene glycol monomethyl ether and 3-phenoxybenzyl alcohol; 1,3-dimethyl-2-imidazolidinone, hexylene glycol, 2,3-butanediol and tetrahydrofuryl alcohol; and 1,3-dimethyl-2-imidazolidinone, 2-phenoxyethanol, diethylene glycol monoethyl ether acetate and 2,3-butanediol.

In an embodiment, examples of organic solvents used in the charge transporting varnish according to the present invention include ethers such as anisole, ethoxybenzene, dimethoxybenzenes and glycol diethers (glycol diethers), such as, ethylene glycol diethers (such as 1,2-dimethoxyethane, 1,2-diethoxyethane, and 1,2-dibutoxyethane); diethylene glycol diethers such as diethylene glycol dimethyl ether, and diethylene glycol diethyl ether; propylene glycol diethers such as propylene glycol dimethyl ether, propylene glycol diethyl ether, and propylene glycol dibutyl ether; dipropylene glycol diethers, such as dipropylene glycol dimethyl ether, dipropylene glycol diethyl ether, and dipropylene glycol dibutyl ether; as well as higher analogues (i.e., tri- and tetra- analogues, e.g., triethylene glycol dimethyl ether, triethylene glycol butyl methyl ether, tetraethylene glycol dimethyl ether, etc.) of the ethylene glycol and propylene glycol ethers mentioned herein.

Still other solvents can be considered, such as ethylene glycol monoether acetates and propylene glycol monoether acetates (glycol ester ethers), wherein the ether can be selected, for example, from methyl, ethyl, n-propyl, isopropyl, n-butyl, sec-butyl, tert-butyl, and cyclohexyl; as well as higher glycol ether analogues of those listed above (such as di-, tri- and tetra-).

Examples include, but are not limited to, propylene glycol methyl ether acetate, 2-ethoxyethyl acetate, 2-butoxyethyl acetate, ethylene glycol monomethyl ether acetate, diethylene glycol monomethyl ether acetate.

Still other solvents such as ethylene glycol diacetate (glycol diesters) can be considered, which include higher glycol ether analogues (such as di-, tri- and tetra-).

Examples include, but are not limited to, ethylene glycol diacetate, triethylene glycol diacetate, propylene glycol diacetate.

Alcohols may also be considered for use in the charge transporting varnish, such as, for example, methanol, ethanol, trifluoroethanol, n-propanol, isopropanol, n-butanol, t-butanol, and alkylene glycol monoethers (glycol monoethers). Examples of suitable glycol monoethers include, but are not limited to, ethylene glycol monopropyl ether, ethylene glycol monohexyl ether (hexyl Cellosolve), propylene glycol monobutyl ether (Dowanol PnB), diethylene glycol monoethyl ether (ethyl Carbitol), dipropylene glycol n-butyl ether (Dowanol DPnB), ethylene glycol monobutyl ether (butyl Cellosolve), diethylene glycol monobutyl ether (butyl Carbitol), dipropylene glycol monomethyl ether (Dowanol DPM), diisobutyl carbinol, 2-ethylhexyl alcohol, methyl isobutyl carbinol, propylene glycol monopropyl ether (Dowanol PnP), diethylene glycol monopropyl ether (propyl Carbitol), diethylene glycol monohexyl ether (hexyl Carbitol), 2-ethylhexyl carbitol, dipropylene glycol monopropyl ether (Dowanol DPnP), tripropylene glycol monomethyl ether (Dowanol TPM), diethylene glycol monomethyl ether (methyl Carbitol), and tripropylene glycol monobutyl ether (Dowanol TPnB).

The organic solvents disclosed herein can be used in varying proportions in the charge transporting varnish, for example, to improve ink characteristics such as substrate wettability, ease of solvent removal, viscosity, surface tension, and jettability.

In an embodiment, the charge transporting varnish comprises dimethyl sulfoxide, ethylene glycol (glycols), tetramethyl urea, or a mixture thereof.

Examples of suitable glycols include, but are not limited to, ethylene glycol, diethylene glycol, dipropylene glycol, polypropylene glycol, propylene glycol, triethylene glycol, and the like.

The above-mentioned glycol diethers, glycol ester ethers, glycol diesters, glycol monoethers, glycol monoethers, glycols, and the like are collectively referred to as "glycol-based solvents." That is, a "glycol-based solvent" as used herein is an organic solvent that does not have one or more aromatic structures represented by the formula $R^{G1}$-O-($R^{G0}$-O)$_v$-$R^{G2}$, wherein each $R^{GO}$ is, independently, a linear $C_2$-$C_4$ unsubstituted alkylene group, and $R^{G1}$ and $R^{G2}$ are each, independently, a hydrogen atom, a linear, branched, or cyclic Ci-Cs unsubstituted alkyl group, or a linear or branched Ci-Cs unsubstituted aliphatic acyl group, and v is an integer of 1 to 6. It is particularly preferable that R is a $C_2$ or $C_3$ unsubstituted alkylene group. As the alkyl group, a linear, branched or cyclic $C_1$-$C_6$ unsubstituted alkyl group is preferable, a linear $C_1$-$C_4$ unsubstituted alkyl group is more preferable, and a methyl group and an n-butyl group are particularly preferable. As the acyl group, a linear or branched $C_2$-$C_6$ unsubstituted aliphatic acyl group is preferable, a linear $C_2$-$C_4$ unsubstituted acyl group is more preferable, and an acetyl group and a propionyl group are particularly preferable. It is particularly preferable that v is an integer of 1 to 4. Such glycol-based solvents include, for example, the following solvents.

- Glycols which are ethylene glycol, propylene glycol or oligomers thereof (dimers to tetramers, e.g. diethylene glycol)
- Glycol monoethers which are monoalkyl ethers of the aforementioned glycols
- Glycol diethers which are dialkyl ethers of the aforementioned glycols
- Glycol monoesters which are aliphatic carboxylic acid monoesters of the aforementioned glycols
- Glycol diesters which are aliphatic carboxylic acid diesters of the aforementioned glycols
- Glycol ester ethers which are aliphatic carboxylic acid monoesters of the aforementioned glycol monoethers

For ease of application by inkjet coating, it is preferable to use a solvent system comprising a glycol-based solvent.

In the following description, glycol-based solvents may be contrasted with organic solvents not falling under this category, and, for convenience, the former may be denoted by (A) and the latter may be denoted by (B).

In an embodiment, the charge transporting varnish is a charge transporting varnish comprising one or more glycol-based solvents (A).

In an embodiment, the charge transporting varnish is a charge transporting varnish comprising one or more glycol-based solvents (A) and one or more organic solvents other than glycol-based solvents (B).

Preferable examples of glycol-based solvents (A) include glycol diethers, glycol monoethers, and glycols, as well as mixtures thereof.

Examples include, but are not limited to, mixtures of a glycol diether and a glycol.

Specific examples include the above-mentioned glycol diethers and glycols; preferable examples of glycol diethers include triethylene glycol dimethyl ether and triethylene glycol butyl methyl ether; and preferable examples of glycols include ethylene glycol and diethylene glycol.

Preferable examples of organic solvents (B) include nitriles, alcohols, aromatic ethers, and aromatic hydrocarbons.

Examples of nitriles include, but are not limited to, methoxypropionitrile and ethoxypropionitrile. Examples of alcohols include, but are not limited to, benzyl alcohol, and 2-(benzyloxy)ethanol. Examples of aromatic ethers include, but are not limited to, methyl anisole, dimethyl anisole, ethyl anisole, butyl phenyl ether, butyl anisole, pentyl anisole, hexyl anisole, heptyl anisole, octyl anisole, phenoxy toluene. Examples of aromatic hydrocarbons include, but are not limited to, pentylbenzene, hexylbenzene, heptylbenzene, octylbenzene, nonylbenzene, cyclohexylbenzene, and tetralin.
Among these, alcohols are more preferable, and 2-(benzyloxy)ethanol is more preferable among the alcohols.
Addition of an organic solvent (B) to a glycol-based solvent (A) allows proper control, at the time of film formation by ink-jet coating, of the aggregation of the metal oxide nanoparticles while maintaining the solubility of the oligoaniline compound and the sulfonic acid ester compound, thereby enabling a flatter film to be formed.
When the organic solvent (B) is added to the glycol-based solvent (A), the amount of glycol-based solvent (A), wtA (in weight), and the amount of organic solvent (B), wtB (in weight), preferably satisfy formula (1-0), more preferably satisfy formula (1-1), even more preferably satisfy formula (1-2), and most preferably satisfy formula (1-3).

$$0.01 \leq \mathrm{wtB} / (\mathrm{wtA} + \mathrm{wtB}) \leq 0.60 \qquad (1\text{-}0)$$

$$0.05 \leq \mathrm{wtB} / (\mathrm{wtA} + \mathrm{wtB}) \leq 0.50 \qquad (1\text{-}1)$$

$$0.10 \leq \mathrm{wtB} / (\mathrm{wtA} + \mathrm{wtB}) \leq 0.40 \qquad (1\text{-}2)$$

$$0.15 \leq \mathrm{wtB} / (\mathrm{wtA} + \mathrm{wtB}) \leq 0.30 \qquad (1\text{-}3)$$

Where the varnish of the present invention comprises two or more glycol-based solvents (A), wtA indicates the total amount (in weight) of the glycol-based solvents (A); where the varnish of the present invention comprises two or more organic solvents (B), wtB indicates the total amount (in weight) of the organic solvents (B).
Further, the charge transporting varnish of the present invention may comprise a silane compound. If the charge transporting varnish comprises a silane compound, use of the charge transporting thin film obtained from the varnish as the hole injection layer of an EL device leads to improvements in, for example, the injectability of holes into the hole transport layer, which is expected to result, for example, in the device having reduced driving voltage and improved durability.
Specific examples of such silane compounds include, for example, alkoxysilane compounds such as a dialkoxysilane compound and a trialkoxysilane compound, and condensates thereof.
In particular, from the perspective of improving the injectability of holes into the hole transport layer when the resulting charge transporting thin film is used as the hole injection layer of an EL device, a fluorinated silane compound is preferable, and a fluorinated alkoxysilane compound is more preferable. Further, from the perspective of reproducibly obtaining a charge transporting thin film having excellent charge transportability, a fluorinated dialkoxysilane or a fluorinated trialkoxysilane is preferable, and a fluorinated trialkoxysilane compound is more preferable.
From the perspective of obtaining the effects mentioned above, the amount of the silane compound is preferably 3% by mass or more based on the total mass of the solids, and from the perspective of reproducibly obtaining a charge transporting thin film having excellent charge transportability, the amount of the silane compound is preferably 20% by mass or less based on the total mass of the solids.
Specific examples of a halogen atom, an alkyl group having 1 to 20 carbon atoms, an alkenyl group having 2 to 20 carbon atoms, an alkynyl group having 2 to 20 carbon atoms, an aryl group having 6 to 20 carbon atoms, and a heteroaryl group having 2 to 20 carbon atoms include the same as those listed above.
Examples of the trialkoxysilane compound include compounds represented by formula (11):

$$Y^1Si(OY^2)_3 \qquad (11)$$

wherein $Y^1$ represents a halogen atom, a hydrogen atom, or an alkyl group having 1 to 20 carbon atoms, an alkenyl group having 2 to 20 carbon atoms, an alkynyl group having 2 to 20 carbon atoms, an aryl group having 6 to 20 carbon atoms or a heteroaryl group having 2 to 20 carbon atoms, each of which may be substituted with Z; $Y^2$ represents an alkyl group having 1 to 20 carbon atoms; wherein Z represents a halogen atom, a haloalkyl group having 1 to 20 carbon atoms, an alkyl group having 1 to 20 carbon atom, a hydroxyl group, a mercapto group, an amino group, a haloalkoxy group having 1 to 20 carbon atoms, an alkoxy group having 1 to 20 carbon atoms, an alkenyl group having 2 to 20

carbon atoms, a haloalkenyl group having 2 to 20 carbon atoms, an alkynyl group having 2 to 20 carbon atoms, a haloalkynyl group having 2 to 20 carbon atoms, an alkylthio group having 1 to 20 carbon atoms, a monoalkylamino group having 1 to 20 carbon atoms, a dialkylamino group having 1 to 20 carbon atoms, a glycidoxy group, an alkylcarbonyl group having 1 to 20 carbon atoms, an alkenylcarbonyl group having 2 to 20 carbon atoms, an alkynylcarbonyl group having 2 to 12 carbon atoms, an alkylcarbonyloxy group having 1 to 12 carbon atoms, an alkenylcarbonyloxy group having 2 to 20 carbon atoms, an alkynylcarbonyloxy group having 2 to 20 carbon atoms, an aryl group having 6 to 20 carbon atoms, a haloaryl group having 6 to 20 carbon atoms, a heteroaryl group having 2 to 20 carbon atoms, or a haloheteroaryl group having 2 to 20 carbon atoms.

Examples of the dialkoxysilane compound include compounds represented by formula (12):

$$Y^3Y^4Si(OY^5)2 \qquad (10)$$

wherein $Y^3$ and $Y^4$ each, independently, represent a halogen atom, or an alkyl group having 1 to 20 carbon atoms, an alkenyl group having 2 to 20 carbon atoms, an alkynyl group having 2 to 20 carbon atoms, an aryl group or a heteroaryl group, each of which may be substituted with Z, and $Y^5$ represents an alkyl group having 1 to 20 carbon atoms, wherein Z means the same as above.

Specific examples of the halogen atom, the alkyl group having 1 to 20 carbon atoms, the alkenyl group having 2 to 20 carbon atoms, the alkynyl group having 2 to 20 carbon atoms, the aryl group having 6 to 20 carbon atoms and the heteroaryl group having 2 to 20 carbon atoms for $Y^1$, $Y^3$, $Y^4$ and Z include the same as those described above.

Among them, $Y^1$ , $Y^3$ and $Y^4$ are preferably an alkyl group having 1 to 20 carbon atoms which may be substituted with Z or an aryl group having 6 to 20 carbon atoms which may be substituted with Z, from the perspective of the availability of silane compounds and the improvement of characteristics of the obtained EL devices.

Specific examples of the haloalkyl group having 1 to 20 carbon atoms include a trifluoromethyl group, a 2,2,2-trifluoroethyl group, a 1,1,2,2,2-pentafluoroethyl group, a 3,3,3-trifluoropropyl group, a 2,2,3,3,3-pentafluoropropyl group, a perfluoropropyl group, a heptafluoroisopropyl group, a perfluorobutyl group, a perfluoropentyl group, a perfluorohexyl group, a perfluoroheptyl group, a perfluorooctyl group, a perfluorononyl group, a perfluorodecyl group, a perfluoroundecyl group, a perfluorododecyl group, heptadecafluoro-1,1,2,2-tetrahydrodecyl, and the like.

Specific examples of the haloalkoxy group having 1 to 20 carbon atoms include a trifluoromethoxy group, a 2,2,2-trifluoroethoxy group, a 1,1,2,2,2-pentafluoroethoxy group, a 3,3,3-trifluoropropoxy group, a 2,2,3,3,3-pentafluoropropoxy group, a perfluoropropoxy group, a heptafluoroisopropoxy group, a perfluorobutoxy group, a perfluoropentyloxy group, a perfluorohexyloxy group, a perfluoroheptyloxy group, a perfluorooctyloxy group, a perfluorononyloxy group, a perfluorodecyloxy group, a perfluoroundecyloxy group, a perfluorododecyloxy group, heptadecafluoro-1,1,2,2-tetrahydrodecyloxy and the like.

Specific examples of the alkoxy group having 1 to 20 carbon atoms include a methoxy group, an ethoxy group, an n-propoxy group, an i-propoxy group, an n-butoxy group, an s-butoxy group, a t-butoxy group, an n-pentoxy group and the like.

Specific examples of the haloalkenyl group having 2 to 20 carbon atoms include a fluorovinyl group, a difluorovinyl group, a 3,3,3-trifluoro-1-propenyl group, a 3,3,3-trifluoro-2-propenyl group, a 2-propenyl group and the like.

Specific examples of the haloalkynyl group having 2 to 20 carbon atoms include a 3,3,3-trifluoro-1-propynyl group, a 3,3,3-trifluoro-2-propynyl group and the like.

Specific examples of the alkylthio group having 1 to 20 carbon atoms include a methylthio group, an ethylthio group, a propylthio group, a butylthio group, a pentylthio group, a hexylthio group, a heptylthio group, an octylthio group, a nonylthio group, a decylthio group, a laurylthio group, and the like.

Specific examples of the monoalkylamino group and dialkylamino group having 1 to 20 carbon atoms include a methylamino group, an ethylamino group, a propylamino group, a butylamino group, a pentylamino group, a hexylamino group, a heptylamino group, an octylamino group, a nonylamino group, a decylamino group, a laurylamino group, a dimethylamino group, a diethylamino group, a dipropylamino group, a dibutylamino group, a dipentylamino group, a dihexylamino group, a diheptylamino group, a dioctylamino group, a dinonylamino group, a didecylamino group and the like.

Specific examples of the alkylcarbonyl group having 1 to 20 carbon atoms include a methylcarbonyl group, an ethylcarbonyl group, an n-propylcarbonyl group, an i-propylcarbonyl group, an n-butylcarbonyl group, an s-butylcarbonyl group, a t-butylcarbonyl group, an n-pentylcarbonyl group and the like.

Specific examples of the alkylcarbonyloxy group having 1 to 20 carbon atoms include a methylcarbonyloxy group, an ethylcarbonyloxy group, an n-propylcarbonyloxy group, an i-propylcarbonyloxy group, an n-butylcarbonyloxy group, an s-butylcarbonyloxy group, a t-butylcarbonyloxy group, an n-pentylcarbonyloxy group and the like.

Specific examples of the alkenylcarbonyl group having 2 to 20 carbon atoms include a vinylcarbonyl group, a 1-propenylcarbonyl group, a 2-propenylcarbonyl group, a 2-methyl-1-propenylcarbonyl group, a 1-methyl-2-propenylcarbonyl group and the like.

Specific examples of the alkynylcarbonyl group having 2 to 20 carbon atoms include an ethynylcarbonyl group, a 1-propynylcarbonyl group, a 2-propynylcarbonyl group, a 2-methyl-1-propynylcarbonyl group, a 1-methyl-2-propynylcarbonyl group and the like.

Specific examples of the alkenylcarbonyloxy group having 2 to 20 carbon atoms include a vinylcarbonyloxy group, a 1-propenylcarbonyloxy group, a 2-propenylcarbonyloxy group, a 2-methyl-1-propenylcarbonyloxy group, a 1-methyl-2-propenylcarbonyloxy group and the like.

Specific examples of the alkynylcarbonyloxy group having 2 to 20 carbon atoms include an ethynylcarbonyloxy group, a 1-propynylcarbonyloxy group, a 2-propynylcarbonyloxy group, a 2-methyl-1-propynylcarbonyloxy group, a 1-methyl-2-propynylcarbonyloxy group and the like.

Specific examples of the haloaryl group having 6 to 20 carbon atoms include a 1-fluorophenyl group, a 2-fluorophenyl group, a 3-fluorophenyl group, a 4-fluorophenyl group, a 5-fluorophenyl group, a pentafluorophenyl group and the like.

Specific examples of the haloheteroaryl group having 2 to 20 carbon atoms include a 3-fluorothiophen-2-yl group, a 4-fluorothiophen-2-yl group, a 5-fluorothiophen-2-yl group and the like.

Of these, from the perspective of the availability of silane compounds and the improvement of characteristics of the resulting EL device, Z is preferably a halogen atom, a haloalkyl group having 1 to 20 carbon atoms, an alky group having 1 to 20 carbon atoms, an alkenyl group having 2 to 20 carbon atoms, a haloalkenyl group having 2 to 20 carbon atoms, an alkynyl group having 2 to 20 carbon atoms, a haloalkynyl group having 2 to 20 carbon atoms, an aryl group having 6 to 20 carbon atoms, a haloaryl group having 6 to 20 carbon atoms, a heteroaryl group having 2 to 20 carbon atoms or a haloheteroaryl group having 2 to 20 carbon atoms; more preferably, a halogen atom, a haloalkyl group having 1 to 20 carbon atoms, an alky group having 1 to 20 carbon atoms, an aryl group having 6 to 20 carbon atoms, or a haloaryl group having 6 to 20 carbon atoms.

In the present invention, a fluorinated silane compound is suitable, as described above.

Accordingly, if the trialkoxysilane mentioned above is used, $Y^1$ is preferably a fluorine atom or a substituent comprising a fluorine atom, and if the dialkoxysilane is used, at least one of $Y^3$ and $Y^4$ is preferably a fluorine atom or a substituent comprising a fluorine atom.

$Y^2$, $Y^5$ and $Y^6$ are alkyl groups having 1 to 20 carbon atoms; from the perspective of the availability of silane compounds and the improvement of characteristics of the resulting EL device, alkyl groups having 1 to 5 carbon atoms are preferable; in particular, methyl groups or ethyl groups are more preferable; methyl groups are even more preferable.

Specific examples of the trialkoxysilane compound include methyltrimethoxysilane, methyltriethoxysilane, ethyltrimethoxysilane, ethyltriethoxysilane, propyltrimethoxysilane, propyltriethoxysilane, butyltrimethoxysilane, butyltriethoxysilane, pentyltrimethoxysilane, pentyltriethoxysilane, heptyltrimethoxysilane, heptyltriethoxysilane, octyltrimethoxysilane, octyltriethoxysilane, dodecyltrimethoxysilane, dodecyltriethoxysilane, hexadecyltrimethoxysilane, hexadecyltriethoxysilane, octadecyltrimethoxysilane, octadecyltriethoxysilane, phenyltrimethoxysilane, phenyltriethoxysilane, vinyltrimethoxysilane, vinyltriethoxysilane, γ-aminopropyltrimethoxysilane, γ-aminopropyltriethoxysilane, γ-glycidoxypropyltrimethoxysilane, γ-glycidoxypropyltriethoxysilane, γ-methacryloxypropyltrimethoxysilane, γ-methacryloxypropyltriethoxysilane, triethoxy(4-(trifluoromethyl)phenyl)silane, dodecyltriethoxysilane, 3,3,3-trifluoropropyltrimethoxysilane, (triethoxysilyl)cyclohexane, perfluorooctylethyltriethoxysilane, triethoxyfluorosilane, tridecafluoro-1,1,2,2-tetrahydroocytyltriethoxysilane, pentafluorophenyl propyl trimethoxysilane, 3-(heptafluoroisopropoxy)propyltriethoxysilane, heptadecafluoro-1,1,2,2-tetrahydrodecyltriethoxysilane, triethoxy-2-thienylsilane, 3-(triethoxysilyl)furan and the like. These may be used alone or in combination of two or more.

Specific examples of preferable trialkoxysilane compounds include methyltrimethoxysilane, methyltriethoxysilane, ethyltrimethoxysilane, ethyltriethoxysilane, propyltrimethoxysilane, propyltriethoxysilane, butyltrimethoxysilane, butyltriethoxysilane, phenyltrimethoxysilane, phenyltriethoxysilane, vinyltrimethoxysilane, vinyltriethoxysilane, γ-glycidoxypropyltrimethoxysilane, γ-glycidoxypropyltriethoxysilane, triethoxy(4-(trifluoromethyl)phenyl)silane, 3,3,3-trifluoropropyltrimethoxysilane, (triethoxysilyl)cyclohexane, perfluorooctylethyltriethoxysilane, triethoxyfluorosilane, tridecafluoro-1,1,2,2-tetrahydrooctyltriethoxysilane, pentafluorophenylpropyltrimethoxysilane, 3-(heptafluoroisopropoxy)propyltriethoxysilane, heptadecafluoro-1,1,2,2-tetrahydrodecyltriethoxysilane, triethoxy-2-thienylsilane, 3-(triethoxysilyl)furan and the like.

Specific examples of more preferable trialkoxysilane compounds include methyltrimethoxysilane, methyltriethoxysilane, ethyltrimethoxysilane, ethyltriethoxysilane, propyltrimethoxysilane, propyltriethoxysilane, butyltrimethoxysilane, butyltriethoxysilane, phenyltrimethoxysilane, phenyltriethoxysilane, vinyltrimethoxysilane, vinyltriethoxysilane, triethoxy(4-(trifluoromethyl)phenyl)silane, 3,3,3-trifluoropropyltrimethoxysilane, (triethoxysilyl)cyclohexane, perfluorooctylethyltriethoxysilane, triethoxyfluorosilane, tridecafluoro-1,1,2,2-tetrahydrooctyltriethoxysilane, pentafluorophenylpropyltrimethoxysilane, 3-(heptafluoroisopropoxy)propyltriethoxysilane, heptadecafluoro-1,1,2,2-tetrahydrodecyltriethoxysilane, triethoxy-2-thienylsilane, 3-(triethoxysilyl)furan and the like.

Specific examples of the dialkoxysilane compound include methylhydrogendimethoxysilane, methylhydrogendiethoxysilane, dimethyldimethoxysilane, dimethyldiethoxysilane, methylethyldimethoxysilane, diethyldimethoxysilane,

diethyldiethoxysilane, methylpropyldimethoxysilane, methylpropyldiethoxysilane, diisopropyldimethoxysilane, phenylmethyldimethoxysilane, vinylmethyldimethoxysilane, γ-glycidoxypropylmethyldimethoxysilane, γ-glycidoxypropylmethyldiethoxysilane, β-(3,4-epoxycyclohexyl)ethylmethyldimethoxysilane, γ-methacryloxypropylmethyldimethoxysilane, γ-methacryloxypropylmethyldiethoxysilane, γ-mercaptopropylmethyldimethoxysilane, γ-aminopropylmethyldiethoxysilane, N-(2-aminoethyl)aminopropylmethyldimethoxysilane, 3,3,3-trifluoropropylmethyldimethoxysilane and the like. These may be used alone or in combination of two or more.

Preferred dialkoxysilane compounds include fluorine atom-containing compounds such as 3,3,3-trifluoropropylmethyldimethoxysilane.

In the charge transporting varnish of the present invention, at least two or more of the silane compounds listed above may be used in combination.

The viscosity of the charge transporting varnish of the present invention is typically 1 to 50 mPa·s at 25°C, and its surface tension is typically 20 to 50 mN/m at 25°C.

The viscosity and surface tension of the charge transporting varnish of the present invention can be adjusted by changing the types of organic solvents used, their ratios, the concentration of the solids and the like, in consideration of various factors such as the method of application to be used and the desired film thickness.

The amount of solids in the charge transporting varnish is preferably 0.001 to 50 wt. %, more preferably 0.01 to 20 wt. %, in consideration of the workability at the time of application of the varnish.

It should be noted that, in the present invention, solids refers to the oligoaniline compound (a), the sulfonic acid ester compound (b), and the metal oxide nanoparticles (c).

In the present invention, the charge transporting varnish is a product formed by dissolving or dispersing, in at least one solvent (d), a charge transporting material comprising the oligoaniline compound (a), which is the main component of the charge transport mechanism, and the sulfonic acid ester compound (b), and further by dispersing the metal oxide nanoparticles (c); from the perspective of obtaining a charge transporting thin film having high flatness reproducibly, the charge transporting varnish is preferably a product formed by dissolving the charge transporting material above in at least one solvent (d) and further dispersing the metal oxide nanoparticles (c).

It should be noted that "charge transporting" is synonymous with "electrically conductive" and that it refers to the ability to transport holes, the ability to transport electrons, or the ability to transport both charges (holes and electrons). The charge transporting varnish according to the present invention may be capable of transporting charges in itself or may be formed into a solid film capable of transporting charges.

The charge transporting varnish described above can be made into a charge transporting thin film on a substrate by applying the charge transporting varnish to a substrate and evaporating the solvent.

The method for varnish application is not specifically restricted, and examples include dipping, spin coating, transfer printing, roll coating, brushing, inkjet coating, and spraying.

The method for solvent evaporation is not specifically restricted, and examples include heating on a hot plate or in an oven in a suitable atmosphere (i.e. under air or an inert gas such as nitrogen, or in a vacuum, etc.). In this way a thin film with a uniform surface can be obtained.

The baking temperature is not specifically restricted so long as it allows the solvent to evaporate, and is preferably 40 to 250°C. Baking may be accomplished in two or more stages at different temperatures for even higher film uniformity or in order to cause reactions to proceed on the substrate.

The charge transporting varnish of the present invention can be produced by mixing the oligoaniline compound, the sulfonic acid ester compound, the metal oxide nanoparticles, and the organic solvent.

The order of mixing is not particularly limited, but examples of methods that allow easy and reproducible production of the charge transporting varnish of the present invention include a method of mixing an oligoaniline compound, a sulfonic acid ester compound, and the like with an organic solvent to obtain a mixture, and then adding to the mixture a pre-prepared sol of metal oxide nanoparticles, or a method of adding this mixture to a pre-prepared sol of metal oxide nanoparticles. In this case, if necessary, an additional organic solvent may be added at the end or some of the components that are relatively soluble in the solvent may be left out of the mixture and then added at the end. From the perspective of suppressing the aggregation and separation of components and reproducibly preparing a charge transporting varnish having excellent uniformity, it is preferable to prepare a well-dispersed or well-dissolved sol of metal oxide nanoparticles in advance separately from the mixture comprising the other components, and then mix the two together, followed by thorough stirring. It should be noted that some of the components of the varnish, especially the metal oxide nanoparticles, may aggregate or precipitate when mixed together, depending on the types and amounts of solvents mixed. It should also be noted that when a varnish is prepared using a sol, the concentration of the sol and the amount of the sol to be used should be determined so that the resulting varnish comprises the desired amount of metal oxide nanoparticles.

The varnish may be heated as appropriate during its preparation as long as the ingredients do not decompose or change in quality.

In the present invention, the charge transporting varnish may be filtered using a sub-micron filter or the like as

an intermediate step during the preparation of the varnish or after having mixed all the components, in order to obtain a thin film having greater flatness reproducibly.

The charge transporting thin film is not specifically restricted in thickness. If it is used as the charge injection layer in an organic EL device, its preferred thickness is 5 to 200 nm. The thickness may be changed by adjusting the concentration of solids in the varnish or the amount of solution on the substrate at the time of application.

The charge transporting thin film obtained as described above comprises metal oxide nanoparticles, and this results in an improved average transmittance (%) over the wavelength range of 400 to 800 nm. As used herein, "improved" means that the average transmittance for a charge transporting thin film comprising metal oxide nanoparticles (c) is greater than that for a corresponding charge transporting thin film without metal oxide nanoparticles (c). The improvement in the average transmittance of the former over the latter is typically 1% or more, preferably 3% or more, and more preferably 5% or more.

As a result, the charge transporting thin film of the present invention has a high light transmittance in the visible region and shows less coloration than conventional ones, despite the fact that an oligoaniline compound (a), a colored material, is used as the charge transporting material. For the charge transporting thin film of the present invention, the average transmittance over the wavelength range of 400 to 800 nm when the film is formed on a quartz substrate at a thickness of 50 nm is typically 90% or more, preferably 95% or more.

The charge transporting varnish of the present invention can be used in the manufacture of either an organic EL device that uses a low molecular light-emitting material (hereinafter referred to as "OLED") or an organic EL device that uses a high molecular light-emitting material (hereinafter referred to as "PLED").

Examples of materials and methods used in the production of an OLED device using the charge transporting varnish of the present invention include, but are not limited to the following.

The electrode substrate to be used should preferably be cleaned beforehand by washing with a liquid such as a detergent, alcohol, and pure water. For example, an anode substrate should preferably undergo surface treatment such as ozone treatment and oxygen-plasma treatment immediately before use. This surface treatment may be omitted if the anode material is composed mainly of organic materials.

The following method may be employed if the charge transporting varnish is to be used in an OLED device.

The charge transporting varnish is applied to the anode substrate. This step is followed by solvent evaporation and baking in the way described above, thereby obtaining a hole transporting thin film (a hole injection layer) formed on the electrode. The electrode is placed in a vacuum evaporation apparatus for sequential deposition thereon of a hole transport layer, a light-emitting layer, an electron transport layer, an electron injection layer, and a cathode metal to obtain an OLED device as desired. A carrier blocking layer may optionally be interposed between any layers to control the light-emitting region.

Examples of the anode material include transparent electrode materials typified by indium tin oxide (ITO) and indium zinc oxide (IZO), and it is preferably planarized in advance. It is also possible to use a polythiophene derivative or a polyaniline derivative having high charge transportability.

Examples of materials used to form the hole transport layer include triarylamines, such as (triphenylamine)dimer derivative (TPD), (a-naphthyldiphenylamine)dimer (a-NPD), and [(triphenylamine)dimer]spirodimer (Spiro-TAD); starburst amines, such as 4,4',4"-tris(3-methylphenyl(phenyl)amino)triphenylamine (m-MTDATA), 4,4',4"-tris[1-naphthyl(phenyl)amino]triphenylamine (1-TNATA); and oligothiophenes, such as 5,5"-bis-{4-[bis(4-methylphenyl)amino]phenyl}-2,2':5',2"-terthiophene (BMA-3T).

Examples of materials used to form the light-emitting layer include tris(8-quinolinolate)aluminum (III) ($Alq_3$), bis(8-quinolinolate)zinc (II) ($Znq_2$), bis(2-methyl-8-quinolinolate)(p-phenylphenolate)aluminum (III) (BAlq), and 4,4'-bis(2,2-diphenylvinyl)biphenyl (DPVBi). The light-emitting layer may be formed by vapor codeposition of an electron transporting material or a hole transporting material together with a light-emitting dopant.

Examples of electron transporting materials include $Alq_3$, BAlq, DPVBi, (2-(4-biphenyl)-5-(4-t-butylphenyl)-1,3,4-oxadiazole) (PBD), triazole derivatives (TAZ), bathocuproine (BCP), and silole derivatives.

Examples of light-emitting dopants include quinacridone, rubrene, coumarin 540, 4-(dicyanomethylene)-2-methyl-6-(p-dimethylaminostylyl)-4H-pyran (DCM), tris(2-phenylpyridine)iridium (III) ($Ir(ppy)_3$), (1,10-phenanthroline)-tris(4,4,4-trifluoro-1-(2-thienyl)-butane-1,3-dionate)europium (III) ($Eu(TTA)_3phen$), and the like.

Examples of materials used to form the carrier blocking layer include PBD, TAZ, BCP, and the like.

Examples of materials used to form the electron injection layer include lithium oxide ($Li_2O$), magnesium oxide (MgO), alumina ($Al_2O_3$), lithium fluoride (LiF), magnesium fluoride ($MgF_2$), strontium fluoride ($SrF_2$), Liq, Li(acac), lithium acetate, lithium benzoate, and the like.

Examples of materials used to form the cathode include aluminum, magnesium-silver alloys, aluminum-lithium alloys, lithium, sodium, potassium, cesium, and the like.

Methods for producing a PLED device using the charge transporting varnish of the present invention are not particularly restricted but include the following exemplary method.

A PLED device comprising a charge transporting thin film (hole injection layer) formed from the charge transporting varnish of the present invention can be produced by forming a light-emitting charge transporting polymer layer instead of performing vacuum deposition to form the hole transport layer, light-emitting layer, electron transport layer, and electron injection layer in the production of an OLED device described above.

Specifically, the PLED device is produced by coating the anode substrate with the charge transporting varnish, thereby forming a hole transporting thin film as described above, and covering it with a light-emitting charge transporting polymer layer and forming a cathode electrode by vapor deposition.

The cathode may be formed from the same materials as used in the production of an OLED device described above. The materials used may undergo cleaning and surface treatment in the same way as described above.

The light-emitting charge transporting polymer layer may be formed by dissolving or uniformly dispersing a light-emitting charge transporting polymer material (alone or together with a light-emitting dopant) in a solvent, applying the resulting solution or dispersion onto an electrode substrate on which a hole injection layer has been formed in advance, and finally evaporating the solvent.

Examples of light-emitting charge transporting polymer materials include, for example, polyfluorene derivatives such as poly(9,9-dialkylfluorene) (PDAF), polyphenylenevinylene derivatives such as poly(2-methoxy-5-(2'-ethylhexoxy)-1,4-phenylenevinylene) (MEH-PPV), polythiophene derivatives such as poly(3-alkylthiophene) (PAT), polyvinylcarbazole (PVCz), and the like.

Examples of solvents include toluene, xylene, chloroform, and the like. Dissolution or uniform dispersion may be accomplished by stirring with or without heating or by ultrasonic dispersion.

Methods of application are not specifically restricted; they include inkjet coating, spraying, dipping, spin coating, transfer printing, roll coating, and brushing. Application should preferably be carried out under an inert gas such as nitrogen and argon.

Solvent evaporation may be accomplished by heating in an oven or on a hot plate under an inert gas or in a vacuum.

The charge transporting varnish of the present invention comprises a sulfonic acid ester compound represented by formula (2) above, in addition to an oligoaniline compound, one or more metal oxide nanoparticles, and an organic solvent. This sulfonic acid ester compound can be stored for a long period of time without precipitation of components and other problems because it is highly soluble in low polarity organic solvents and, at the same time, has excellent stability.

In other words, in the present invention, use of a sulfonic acid ester compound represented by formula (2) together with an oligoaniline compound, one or more metal oxide nanoparticles, and an organic solvent, particularly a low polarity organic solvent, as components of a charge transporting varnish, allows the storage stability of the varnish to be improved, and avoids or lessens problems such as, for example, precipitation or deposition of components or degradation of electrical properties of the film obtained from the varnish that might occur after storage under air.

**Examples**

Hereinafter, the present invention will be described more specifically with reference to manufacturing examples, preparation examples and examples, but the present invention is not limited to the examples described below. The apparatus used were as follows.

(1) Substrate cleaning: Substrate cleaning equipment (reduced-pressure plasma system), manufactured by Choshu Industry Co., Ltd.
(2) Varnish application: MS-A100 spin coater, manufactured by Mikasa Co., Ltd.
(3) Film thickness measurement: Microfigure measuring instrument surfcorder ET-4000, manufactured by Kosaka Laboratory, Ltd.
(4) EL device fabrication: C-E2L1G1-N multifunction evaporation system, manufactured by Choshu Industry Co., Ltd.
(5) Measurement of EL device luminance etc.: Multi-channel IVL measuring system, manufactured by EHC Co., Ltd.
(6) Measurement of EL device lifetime (half-life measurement): PEL-105S organic EL luminance lifetime evaluation system, manufactured by EHC Co., Ltd.
(7) Measurement of transmittance: UV-3600 ultraviolet-visible-near-infrared spectrophotometer from Shimadzu Science.

**Manufacturing Example 1**

Arylsulfonic acid ester (S1) was synthesized in accordance with the following scheme.

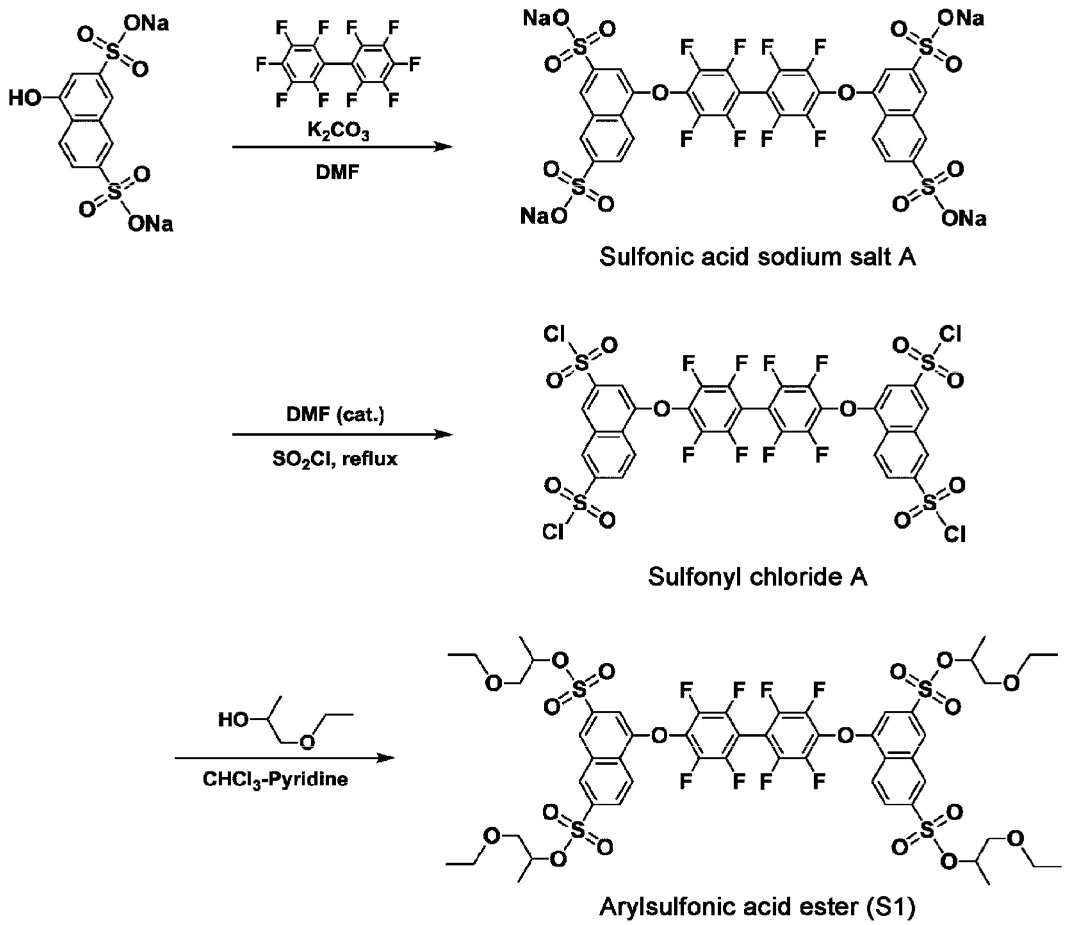


Sulfonic acid sodium salt A

Sulfonyl chloride A

Arylsulfonic acid ester (S1)

Under a nitrogen atmosphere, 4.8 g (14.36 mol) of perfluorobiphenyl, 4.2 g (30.15 mol) of potassium carbonate and 100 mL of N,N-dimethylformamide were successively added to 11 g (31.59 mmol) of sodium 1-naphthol-3,6-disulfonate, and the reaction system was flushed with nitrogen and subsequently stirred for 6 hours at an internal temperature of 100°C. The system was allowed to cool to room temperature, following which the potassium carbonate residue was removed by filtration and vacuum concentration was carried out. To remove the remaining impurities, 100 mL of methanol was added to the residue and was stirred for 30 minutes at room temperature. The suspension was then filtered to give 11.8 g (yield: 83%) of sulfonic acid sodium salt A.

Thionyl chloride (8 mL) and DMF (0.1 mL) were added to 2 g (2 mmol) of sulfonic acid sodium salt A and the system was refluxed under heating for one hour, following which the thionyl chloride was driven off to give a solid containing sulfonyl chloride A. This compound was used in the next step without further purification.

Chloroform (12 mL) and pyridine (8 mL) were added to this solid, and 2.50 g (24 mmol) of propylene glycol monoethyl ether (Junsei Chemical Co., Ltd.) was added at 0°C. The mixture was brought to room temperature and then stirred for 3 hours. The solvent was driven off, following which water was added, extraction was carried out with ethyl acetate, and the organic layer was dried over sodium sulfate. After filtration and concentration, the resulting crude product was purified by silica gel column chromatography (hexane/ethyl acetate) to give 1.09 g of arylsulfonic acid ester (S1) as a white solid (yield: 44% (2-step yield from sulfonic acid sodium salt A)). The results of $^1$H-NMR and LC/MS measurements are shown below. The arylsulfonic acid ester (S1) obtained was used for the preparation of the varnishes described below.

$^1$H-NMR(500 MHz, $CDCl_3$): δ 0.92-0.97 (m, 12H), 1.34 and 1.40 (a pair of d, J = 6.5 Hz, 12H), 3.32-3.52 (m, 16H), 4.80-4.87 (m, 4H), 7.37 (s, 2H), 8.22 (d, J = 8.5 Hz, 2H), 8.45 (s, 2H), 8.61 (d, J = 8.5 Hz, 2H), 8.69 (s, 2H).
LC/MS (ESI$^+$) m/z; 1264 $[M+NH_4]^+$

**Preparation Example 1**

A 500 ml eggplant-shaped flask was charged with 250 g of a silica sol dispersed in MEK (MEK-ST, manufactured by Nissan Chemical Industries, Ltd., particle size: 10-15 nm, $SiO_2$: 30 wt. %) and 170 g of triethylene glycol butyl methyl ether, and was set in a rotary evaporator. The mixture was concentrated in vacuo to a weight of 250 g to obtain a silica

sol dispersed in triethylene glycol butyl methyl ether ($SiO_2$: 30 wt. %). The silica sol dispersed in triethylene glycol butyl methyl ether obtained was used for the preparation of the varnishes described below.

## [1] Varnish Preparation

### Example 1-1

Under a nitrogen atmosphere, 0.068 g of the oligoaniline compound represented by formula (A1) above and 0.248 g of arylsulfonic acid ester (S1) were dissolved in a mixed solvent of 4.52 g of triethylene glycol butyl methyl ether, 3.0 g of butyl benzoate, and 2.0 g of dimethyl phthalate. To this, 0.690 g of the silica sol dispersed in triethylene glycol butyl methyl ether was added and stirred to obtain a charge transporting varnish. The oligoaniline compound represented by formula (A1) above (hereinafter referred to as "oligoaniline compound (A1)") was synthesized according to the method described in WO 2013/084664.

### Example 1-2

Under a nitrogen atmosphere, 0.045 g of oligoaniline compound (A1) and 0.165 g of arylsulfonic acid ester (S1) were dissolved in a mixed solvent of 4.28 g of triethylene glycol butyl methyl ether, 3.0 g of butyl benzoate, and 2.0 g of dimethyl phthalate. To this, 1.035 g of the silica sol dispersed in triethylene glycol butyl methyl ether was added and stirred to obtain a charge transporting varnish.

### Example 1-3

Under a nitrogen atmosphere, 0.023 g of oligoaniline compound (A1) and 0.083 g of arylsulfonic acid ester (S1) were dissolved in a mixed solution of 4.04 g of triethylene glycol butyl methyl ether, 3.0 g of butyl benzoate, and 2.0 g of dimethyl phthalate. To this, 1.381 g of the silica sol dispersed in triethylene glycol butyl methyl ether was added and stirred to obtain a charge transporting varnish.

### Comparative Example 1-1

An attempt was made to prepare a charge transporting varnish in the same way as in Example 1-1, except that 0.205 g of the arylsulfonic acid represented by formula (S4) below was used in place of arylsulfonic acid ester (S1), but it was not possible to prepare a charge transporting varnish sufficiently homogeneous for use in the manufacture of a charge transporting thin film, because the arylsulfonic acid compound represented by formula (S4) below did not dissolve. The arylsulfonic acid represented by formula (S4) below (hereinafter referred to as "arylsulfonic acid (S4)") was synthesized according to the method described in WO 2006/025342 (the same applies hereinafter).

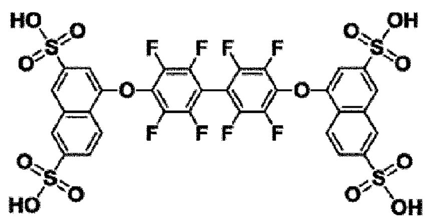

(S4)

### Comparative Example 1-2

Under a nitrogen atmosphere, 0.104 g of oligoaniline compound (A1) and 0.205 g of arylsulfonic acid (S4) were dissolved in 3.3 g of 1,3-dimethyl-2-imidazolidinone. To this, 4.0 g of 2,3-butanediol and 2.7 g of dipropylene glycol monomethyl ether were added and stirred to obtain a charge transporting varnish.

### Comparative Example 1-3

Under a nitrogen atmosphere, 0.114 g of oligoaniline compound (A1) and 0.413 g of arylsulfonic acid ester (S1) were dissolved in a mixed solvent of 5.0 g of triethylene glycol butyl methyl ether, 3.0 g of butyl benzoate and 2.0 g of dimethyl phthalate to obtain a charge transporting varnish.

**Example 4-1**

Under a nitrogen atmosphere, 0.045 g of oligoaniline compound (A1), 0.058 g of 3,3,3-trifluoropropyltrimethoxysilane (manufactured by Shin-Etsu Chemical Co., Ltd.), and 0.165 g of arylsulfonic acid ester (S1) were dissolved in a mixed solvent of 4.59 g of triethylene glycol butyl methyl ether, 3.32 g of butyl benzoate, and 2.21 g of dimethyl phthalate. To this, 1.033 g of the silica sol dispersed in triethylene glycol butyl methyl ether was added and stirred to obtain a charge transporting varnish.

**Example 4-2**

Under a nitrogen atmosphere, 0.037 g of oligoaniline compound (A1), 0.007 g of the oligoaniline compound represented by formula (A2) above (hereinafter referred to as "oligoaniline compound (A2)"), and 0.166 g of arylsulfonic acid ester (S1) were dissolved in a mixed solvent of 4.27 g of triethylene glycol butyl methyl ether, 2.99 g of butyl benzoate, and 1.95 g of dimethyl phthalate. To this, 1.033 g of the silica sol dispersed in triethylene glycol butyl methyl ether was added and stirred to obtain a charge transporting varnish. Oligoaniline compound (A2) was synthesized according to the method described in Synthesis Example 1 of WO 2016/190326 (the same applies hereinafter).

**Example 4-3**

Under a nitrogen atmosphere, 0.034 g of oligoaniline compound (A1), 0.023 g of the oligoaniline compound represented by formula (A3) above (hereinafter referred to as "oligoaniline compound (A3)"), and 0.154 g of arylsulfonic acid ester (S1) were dissolved in a mixed solvent of 4.27 g of triethylene glycol butyl methyl ether, 2.99 g of butyl benzoate acid, and 1.99 g of dimethyl phthalate. To this, 1.033 g of the silica sol dispersed in triethylene glycol butyl methyl ether was added and stirred to obtain a charge transporting varnish. Oligoaniline compound (A3) was synthesized according to the method described in Production Example 24-2 of WO 2015/050253.

**[2] Evaluation of transmittance**

**Example 2-1**

The varnish obtained in Example 1-1 was applied to a quartz substrate using a spin coater, and then dried by baking in an air atmosphere at 120°C for 1 minute. Next, the dried quartz substrate was baked at 230°C for 15 minutes in an air atmosphere to form a uniform thin film of 50 nm thickness on the quartz substrate.

**Examples 2-2 to 2-3 and Comparative Example 2-2**

Thin films were formed according to the same method as in Example 2-1, except that the varnish obtained in Example 1-2, Example 1-3 or Comparative Example 1-3, respectively, was used in place of the varnish obtained in Example 1-1.

**Comparative Example 2-1**

A thin film was formed according to the same method as in Example 2-1, except that the varnish obtained in Comparative Example 1-2 was used in place of the varnish obtained in Example 1-1 and the drying temperature was set at 80°C.

Light transmittance was measured using a spectrophotometer for the quartz substrates carrying thin films of 50 nm thickness obtained in Examples 2-1 to 2-3 and Comparative Examples 2-1 to 2-2. The results are shown in FIG. 1 and Table 1.

Table 1

| | Average transmittance (%) from 400 nm to 800 nm |
|---|---|
| Example 2-1 | 93.8 |
| Example 2-2 | 97.0 |
| Example 2-3 | 99.2 |
| Comparative Example 2-1 | 90.3 |

(continued)

| | Average transmittance (%) from 400 nm to 800 nm |
|---|---|
| Comparative Example 2-2 | 90.0 |

As shown in FIG. 1 and Table 1, the addition of an organosilica sol improved the average light transmittance in the visible region.

In other words, the charge transporting thin films of the present invention comprising silica particles showed superior transmittance in the visible region, compared to the thin films comprising no silica particles.

**[3] Fabrication and characterization of organic EL devices**

**Example 3-1**

The varnish obtained in Example 1-1 was applied to an ITO substrate using a spin coater, and was subsequently dried at 120°C under air atmosphere for 1 minute. Subsequently, the dried ITO substrate was baked at 230°C for 15 minutes under air atmosphere to form a uniform thin film of 50 nm thickness on the ITO substrate. For the ITO substrate, a glass substrate (25 mm × 25 mm × 0.7t) on the surface of which a patterned indium tin oxide (ITO) film having a thickness of 150 nm was formed was used, and the impurities on the surface of the glass substrate were removed by an $O_2$ plasma cleaning device (150 W, 30 seconds) prior to use.

Next, α-NPD (N,N'-di(1-naphthyl)-N,N'-diphenylbenzidine) was deposited using a vacuum evaporator (degree of vacuum: 1.0 × $10^{-5}$ Pa) to a thickness of 30 nm at a rate of 0.2 nm/s on the ITO substrate on which the thin film had been formed. Subsequently, another film of HTEB-01 (an electron blocking material manufactured by Kanto Chemical Co., Ltd.) was formed to a thickness of 10 nm. Then, NS60 (a host material for the light emitting layer manufactured by Nippon Steel & Sumikin Chemical Co., Ltd.) and $Ir(PPy)_3$ (a dopant material for the light emitting layer) were co-deposited. In this co-deposition process, the deposition rate was controlled so that the concentration of $Ir(PPy)_3$ was 6%, and the layer was deposited to a thickness of 40 nm. Next, thin films of $Alq_3$, lithium fluoride and aluminum were successively deposited to obtain an organic EL device. In these steps, the deposition rates were set to 0.2 nm/s for $Alq_3$, 0.2 nm/s for aluminum, and 0.02 nm/s for lithium fluoride and the film thicknesses were set to 20 nm, 0.5 nm, and 80 nm, respectively.

In order to prevent deterioration of characteristic under the influence of, among other things, oxygen and water in the air, the organic EL device was sealed using sealing substrates before the characteristics thereof were evaluated. Sealing was performed by the following procedure. The organic EL device was placed between sealing substrates in a nitrogen atmosphere having an oxygen concentration of 2 ppm or less and a dew point of -76°C or less, and the sealing substrates were bonded to each other using an adhesive (Moresco Moisture Cut WB90US (P), manufactured by MORESCO Corporation). In this step, a water-trapping agent (HD-071010W-40, manufactured by Dynic Corporation) was placed between the sealing substrates together with the organic EL device, and the sealing substrates stuck together were irradiated with UV light (wavelength: 365 nm, irradiation level: 6,000 mJ/$cm^2$), and then annealed at 80°C for 1 hour to cure the adhesive.

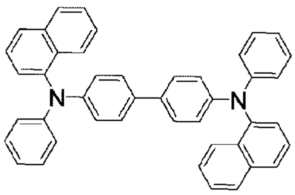

NPB

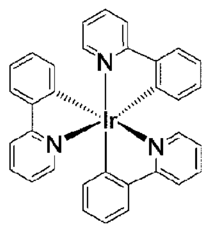

$Ir(PPy)_3$

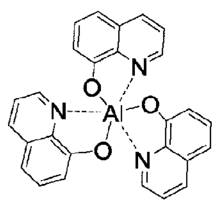

$Alq_3$

**Examples 3-2 to 3-3 and Comparative Example 3-2**

Organic EL devices were obtained in the same manner as in Example 3-1, except that the varnish obtained in Example 1-2, Example 1-3, or Comparative Example 1-3, respectively, was used instead of the varnish obtained in Example 1-1.

**Comparative Example 3-1**

An organic EL device was obtained in the same manner as in Example 3-1, except that the varnish obtained in Comparative Example 1-2 was used instead of the varnish obtained in Example 1-1 and the drying temperature was set at 80°C.

**Example 5-1 to Example 5-3**

Organic EL devices were obtained in the same manner as in Example 3-1, except that the varnishes obtained in Examples 4-1 to 4-3, respectively, were used instead of the varnish obtained in Example 1-1.
The devices obtained were driven at a luminance of 10,000 cd/$m^2$, and their drive voltage, current density, and luminous efficacy, and luminance half-life (time elapsed before the luminance, from an initial value of 10,000 cd/$m^2$, reaches half the initial value) were measured. The results are shown in Table 2.

Table 2

| | Drive voltage (V) | Current density (mA/$cm^2$) | Current efficiency (cd/A) | External quantum efficiency (%) | Luminance half-life (h) |
|---|---|---|---|---|---|
| Example 3-1 | 6.4 | 21.3 | 47.0 | 13.5 | 474 |
| Example 3-2 | 6.3 | 20.9 | 48.0 | 13.7 | 546 |
| Example 3-3 | 6.8 | 20.4 | 49.2 | 14.1 | 567 |
| Comparative Example 3-1 | 6.3 | 22.2 | 45.1 | 12.9 | 502 |
| Comparative Example 3-2 | 6.6 | 22.6 | 44.3 | 12.7 | 383 |
| Example 5-1 | 6.0 | 20.5 | 48.9 | 14.0 | 568 |
| Example 5-2 | 6.0 | 20.4 | 49.1 | 14.1 | 557 |
| Example 5-3 | 6.0 | 20.3 | 49.3 | 14.2 | 576 |

As can be seen from Comparative Example 1-1, when arylsulfonic acid (S4) was used, it was not possible to obtain a charge transporting varnish with the solvent used in the present invention.
However, when arylsulfonic acid ester (S1) was used in place of arylsulfonic acid (S4), the oligoaniline compound and sulfonic acid ester compound were uniformly dissolved and the silica particles, which are metal oxide nanoparticles, were uniformly dispersed, resulting in a charge transporting varnish having excellent storage stability.
As shown in Table 2, the organic EL devices comprising the charge transporting thin films obtained from the charge transporting varnishes of the present invention showed improved current efficiencies as a result of the addition of the organosilica sol. In other words, the presence of silica particles in the charge transporting thin films of the organic EL devices as described above resulted in the thin films having high transparency, thereby leading to improved light extraction efficiency. They also showed excellent lifetime performance.

**[4] Evaluation of varnish storage stability**

**Example 6-1**

A uniform 50 nm thin film was formed on an ITO substrate using the same method as in Example 2-2, except that an ITO substrate was used in place of the quartz substrate.
Next, a 30 nm film of α-NPD was formed at a rate of 0.2 nm/s on the ITO substrate on which the thin film had been formed using a vacuum evaporator (degree of vacuum: $1.0 \times 10^{-5}$ Pa). Subsequently, a hole-only device (HOD device) was obtained by depositing an 80 nm layer of aluminum under the condition of 0.2 nm/s. Sealing was carried out in the same way as in Example 3-1. Then, current density was measured for when a voltage of 3 V was applied to the device obtained (i.e. evaluation of the varnish prior to storage).
The varnish obtained in Example 1-2 was stored at room temperature and under air for one month. A device was then fabricated and evaluated in the same manner as for the evaluation of the varnish prior to storage, except that

the varnish that had been stored was used instead of the varnish prior to storage (i.e. evaluation of the varnish after storage).

**Comparative Example 6-1**

Evaluations of a varnish prior to storage and a varnish after storage were carried out in the same manner as in Example 6-1, except that the varnish obtained in Comparative Example 1-2 was used in place of the varnish obtained in Example 1-2 and the drying temperature was set at 80°C.

The rate of change in current density was calculated by the following equation: (current density in the evaluation of the varnish after storage) / (current density in the evaluation of the varnish prior to storage).

Table 3

| | Rate of change in current density |
|---|---|
| Example 6-1 | 1.2 |
| Comparative Example 6-1 | 0.5 |

As is evident in Table 3, when the varnishes were stored under air at room temperature for one month, the charge transporting varnish of the comparative example showed a significant decrease in the current density of the HOD device comprising the resulting thin film, whereas the charge transporting varnish of the present invention did not show such a decrease.

## Claims

**1.** A charge transporting varnish comprising (a) to (d) below:

(a) an oligoaniline compound;
(b) a sulfonic acid ester compound represented by formula (2) below:

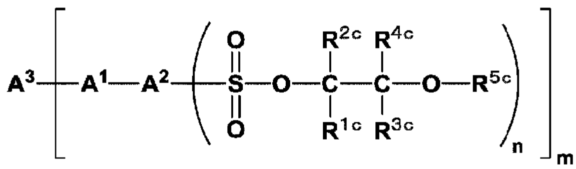

(2)

wherein

$R^{1c}$ to $R^{4c}$ each, independently, represent a hydrogen atom or a linear or branched alkyl group having 1 to 6 carbon atoms,
$R^{5c}$ represents an optionally substituted monovalent hydrocarbon group having 2 to 20 carbon atoms,
$A^1$ represents -O- or -S-,
$A^2$ represents an (n+1)-valent group derived from naphthalene or anthracene,
$A^3$ represents an m-valent group derived from perfluorobiphenyl,
m represents an integer satisfying $2 \leq m \leq 4$, and
n represents an integer satisfying $1 \leq n \leq 4$;

(c) one or more metal oxide nanoparticles; and
(d) an organic solvent.

**2.** The charge transporting varnish according to claim 1, wherein the oligoaniline compound (a) is at least one selected from the group consisting of (i) to (v) below:

(i) an oligoaniline compound represented by formula (1a) below:

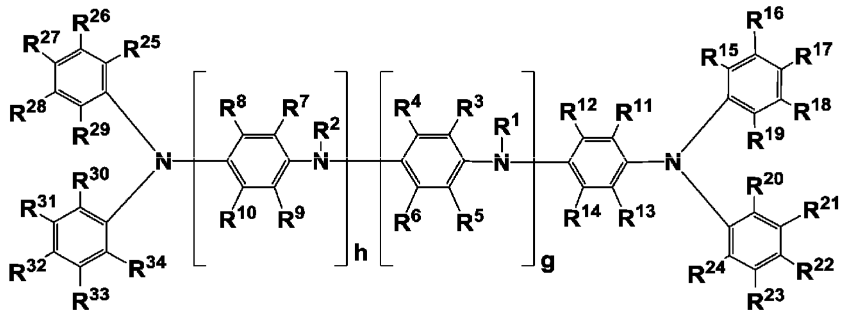

(1a)

wherein

$R^1$ and $R^2$ each, independently, represent a hydrogen atom, a substituted or unsubstituted monovalent hydrocarbon group, a t-butoxycarbonyl group, or a benzyloxycarbonyl group;
$R^3$ to $R^{34}$ each, independently, represent a hydrogen atom, a hydroxyl group, a silanol group, a thiol group, a carboxyl group, a phosphoric acid group, a phosphate ester group, an ester group, a thioester group, an amide group, a nitro group, a substituted or unsubstituted monovalent hydrocarbon group, an organoxy group, an organoamino group, an organosilyl group, an organothio group, an acyl group, a sulfo group or a halogen atom; and
g and h each, independently, represent an integer of 1 or more and satisfy $g + h \leq 20$;

(ii) an oligoaniline compound represented by formula (1b) below:

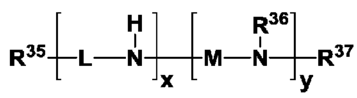

(1b)

wherein

$R^{35}$, $R^{36}$, and $R^{37}$ each, independently, represent a hydrogen atom, an unsubstituted or substituted monovalent hydrocarbon group, or an organoxy group;
L and M each, independently, represent a divalent group represented by formula (b-1) or (b-2) below:

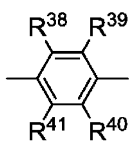

(b-1)

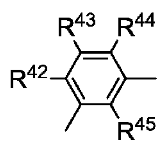

(b-2)

wherein
$R^{38}$ to $R^{45}$ each, independently, represent a hydrogen atom, a hydroxyl group, an unsubstituted or substituted monovalent hydrocarbon group, or an organoxy group, an acyl group, or a sulfonic acid group; and
x and y each, independently, represent an integer of 1 or more, and satisfy $x + y \leq 20$;

(iii) an oligoaniline compound represented by formula (1c) below:

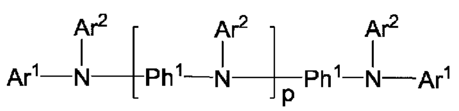

(1c)

wherein
$Ph^1$ is a group represented by formula (PI) below:

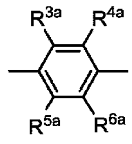

(P1)

wherein

$R^{3a}$ to $R^{6a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^1$ each, independently, represents an unsubstituted or substituted aryl group or heteroaryl group which may have a monoarylamino group and/or a diarylamino group;
$Ar^2$ each, independently, represents an unsubstituted or substituted aryl group or heteroaryl group which may have a monoarylamino group and/or a diarylamino group; and
p represents an integer of 1 to 10;

(iv) an oligoaniline compound represented by formula (1d) below:

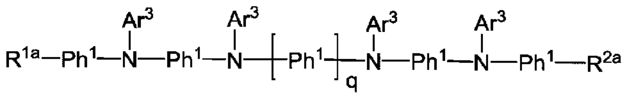

(1d)

wherein

$R^{1a}$ and $R^{2a}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
$Ar^3$ each, independently, represents a diarylaminophenyl group;
q represents 1 or 2; and
$Ph^1$ is the same as defined for formula (1c) above; and

(v) an oligoaniline compound represented by formula (1e) below:

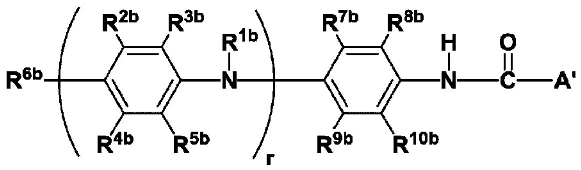

(1e)

wherein

$R^{1b}$ represents a hydrogen atom or an alkyl group having 1 to 20 carbon atoms optionally substituted with $Z^b$;
$Z^b$ represents a halogen atom, a nitro group, a cyano group, an aldehyde group, a hydroxyl group, a thiol group, a sulfonic acid group, a carboxyl group, an aryl group having 6 to 20 carbon atoms optionally substituted with $Z^{b'}$, or a heteroaryl group having 2 to 20 carbon atoms optionally substituted with $Z^{b'}$;
$Z^{b'}$ represents a halogen atom, a nitro group, a cyano group, an aldehyde group, a hydroxyl group, a thiol group, a sulfonic acid group or a carboxyl group;
$R^{2b}$ to $R^{10b}$ each, independently, represent a hydrogen atom, a halogen atom, a nitro group, a cyano group, or an alkyl group having 1 to 20 carbon atoms, an alkenyl group having 2 to 20 carbon atoms, an alkynyl group having 2 to 20 carbon atoms, an aryl group having 6 to 20 carbon atoms, or a heteroaryl group having 2 to 20 carbon atoms optionally substituted with a halogen atom;
A' represents a hydrocarbon group having 1 to 40 carbon atoms, wherein at least one hydrogen atom is

substituted with a fluorine atom and at least one other hydrogen atom is substituted with another atom or substituent; and
r is an integer from 1 to 20.

**3.** The charge transporting varnish according to claim 1 or 2, further comprising at least one silane compound.

**4.** A charge transporting thin film produced from the charge transporting varnish according to any one of claims 1 to 3.

**5.** An organic electroluminescent device comprising the charge transporting thin film according to claim 4.

**6.** A method for producing a charge transporting thin film, comprising the step of applying a charge transporting varnish according to any one of claims 1 to 3 onto a substrate and evaporating the solvent.

**7.** A method for producing the charge transporting varnish according to claim 1, wherein a sol of the one or more metal oxide nanoparticles (c) is used.

**8.** A method for improving the storage stability of a charge transporting varnish comprising an oligoaniline compound, one or more metal oxide nanoparticles, a precursor of an electron accepting substance, and an organic solvent, wherein a sulfonic acid ester compound represented by formula (2) below is used as a precursor of the electron accepting substance.

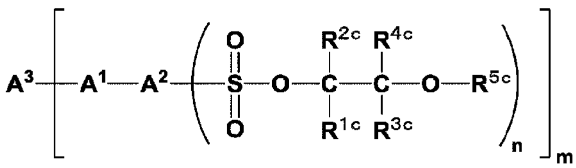

(2)

wherein

$R^{1c}$ to $R^{4c}$ each, independently, represent a hydrogen atom or a linear or branched alkyl group having 1 to 6 carbon atoms,
$R^{5c}$ represents an optionally substituted monovalent hydrocarbon group having 2 to 20 carbon atoms,
$A^1$ represents -O- or -S-,
$A^2$ represents an (n+1)-valent group derived from naphthalene or anthracene,
$A^3$ represents an m-valent group derived from perfluorobiphenyl,
m represents an integer satisfying $2 \leq m \leq 4$, and
n represents an integer satisfying $1 \leq n \leq 4$.

FIG. 1

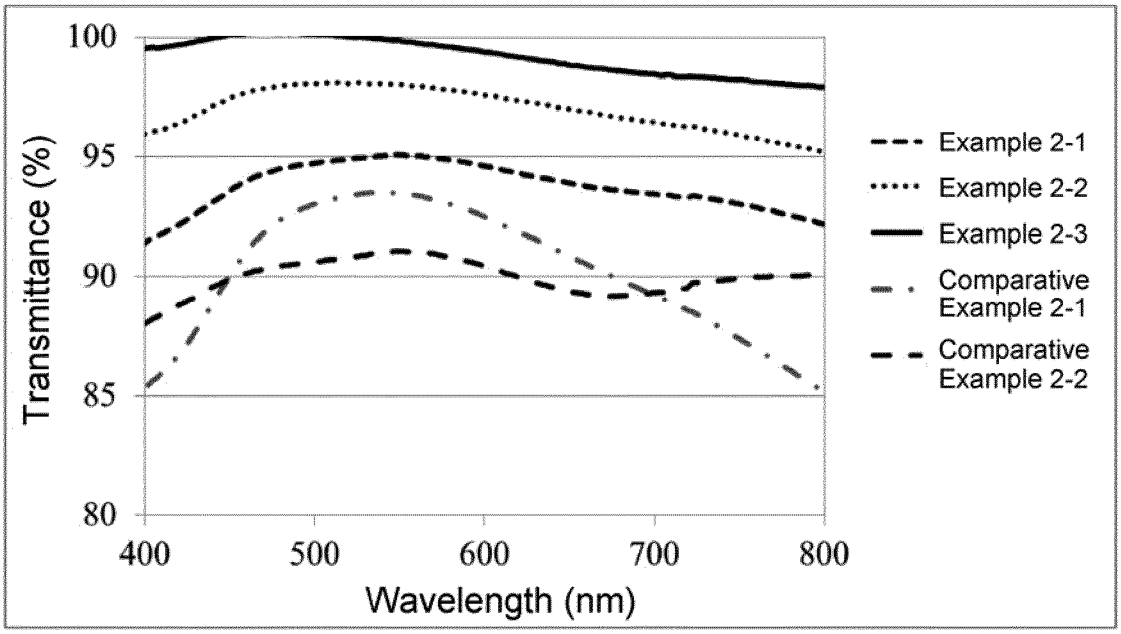
100
95
90
85
80
Transmittance (%)
400
500
600
700
800
Wavelength (nm)
Example 2-1
Example 2-2
Example 2-3
Comparative Example 2-1
Comparative Example 2-2

## INTERNATIONAL SEARCH REPORT

International application No.
PCT/JP2018/046690

A. CLASSIFICATION OF SUBJECT MATTER
Int.Cl. H01L51/50(2006.01)i, C09D7/61(2018.01)i, C09D7/63(2018.01)i, C09D179/02(2006.01)i, H05B33/10(2006.01)i

According to International Patent Classification (IPC) or to both national classification and IPC

B. FIELDS SEARCHED

Minimum documentation searched (classification system followed by classification symbols)
Int.Cl. H01L51/50, C09D7/61, C09D7/63, C09D179/02, H05B33/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2007/099808 A1 (NISSAN CHEMICAL INDUSTRIES, LTD.) 07 September 2007, paragraphs [0001]-[0010], [0056]-[0064], [0082]-[0117], claims & TW 200800870 A & JP 5136795 B2 | 1-8 |
| A | JP 2007-137801 A (FUJI XEROX CO., LTD.) 07 June 2007, claims, paragraphs [0008], [0041]-[0045], [0257] (Family: none) | 1-8 |
| A | WO 2015/050253 A1 (NISSAN CHEMICAL INDUSTRIES, LTD.) 09 April 2015, paragraphs [0005], [0155]-[0180], [0223], [0233]-[0330], [0344]-[0345] & US 2016/0301011 A1 pargaraphs [0011], [0162]-[0196], [0278], [0302]-[0567], [0581] & EP 3053910 A1 & KR 10-2016-0067925 A & CN 105793233 A & TW 201524955 A | 1-8 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 March 2019 (18.03.2019) | 26 March 2019 (26.03.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/046690

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/190326 A1 (NISSAN CHEMICAL INDUSTRIES, LTD.) 01 December 2016, paragraphs [0007]-[0146] & US 2018/0151805 A1 paragraphs [0010]-[0163] & EP 3306692 A1 & KR 10-2017-0049556 A & CN 107112428 A | 1-8 |
| A | WO 2013/084664 A1 (NISSAN CHEMICAL INDUSTRIES, LTD.) 13 June 2013, paragraph [0068], claims & US 2015/0008372 A1 paragraph [0107], claims & EP 2789668 A1 & CN 103975038 A & KR 10-2014-0113930 A & TW 201335115 A | 1-8 |
| A | JP 2007-531802 A (E.I. DU PONT DE NEMOURS AND COMPANY) 08 November 2007, entire text & US 2004/0206942 A1 & WO 2005/093872 A1 & WO 2004/029176 A1 & EP 1546283 A1 & KR 10-2007-0004857 A & CN 1934725 A & TW 200539195 A & CA 2500304 A | 1-8 |
| A | JP 2009-290204 A (DAINIPPON PRINTING CO., LTD.) 10 December 2009, entire text & US 2011/0163327 A1 & WO 2009/133907 A1 & EP 2276087 A1 & KR 10-2011-0008196 A & CN 102017217 A | 1-8 |
| A | JP 2010-034315 A (IDEMITSU KOSAN CO., LTD.) 12 February 2010, entire text (Family: none) | 1-8 |
| A | JP 2012-023020 A (RICOH CO., LTD.) 02 February 2012, entire text & US 2011/0309347 A1 & EP 2398087 A1 & CN 102364716 A | 1-8 |
| P, A | WO 2018/135580 A1 (NISSAN CHEMICAL INDUSTRIES, LTD.) 26 July 2018, entire text (Family: none) | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002151272 A
- WO 2004043117 A
- WO 2005043962 A
- WO 2005000832 A
- JP H07134416 B
- JP 5136795 B
- WO 2008129947 A
- WO 2006025342 A
- WO 2013084664 A
- WO 2016190326 A
- WO 2015050253 A


### Non-patent literature cited in the description


- *Chemische Berichte,* 1957, vol. 90, 585-592
- *Kino Zairyo,* 2004, vol. 24, 72-82